(19) Europäisches Patentamt
European Patent Office
Office européen des brevets

(11) Veröffentlichungsnummer: **0 281 121 B1**

(12) **EUROPÄISCHE PATENTSCHRIFT**

(45) Veröffentlichungstag der Patentschrift: **29.04.92**

(51) Int. Cl.[5]: **C07D 233/60**, A61K 31/415

(21) Anmeldenummer: **88103225.4**

(22) Anmeldetag: **03.03.88**

# (54) 1-(1-Aryl-2-hydroxy-ethyl)-imidazole und deren Salze, Verfahren zu ihrer Herstellung, diese Verbindungen enthaltende Arzneimittel und ihre Verwendung.

(30) Priorität: **06.03.87 DE 3707151**

(43) Veröffentlichungstag der Anmeldung:
**07.09.88 Patentblatt 88/36**

(45) Bekanntmachung des Hinweises auf die Patenterteilung:
**29.04.92 Patentblatt 92/18**

(84) Benannte Vertragsstaaten:
**AT BE CH DE ES FR GB GR IT LI LU NL SE**

(56) Entgegenhaltungen:
**EP-A- 0 025 948**
**EP-A- 0 218 118**

**CHEMICAL ABSTRACTS, Band 91, Nr. 7, 13. August 1979, Columbus, Ohio, US; ROHM AND HAAS CO: "1-(2-Aryl-2-hydroxyethyl)imidazoles." Seite 709, Spalte 2, Zusammenfassung Nr. 57 005p**

(73) Patentinhaber: **HOECHST AKTIENGESELLSCHAFT**
**Postfach 80 03 20**
**W-6230 Frankfurt am Main 80(DE)**

(72) Erfinder: **Kampe, Klaus-Dieter, Dr.**
**Am Rehsteig 1**
**W-6232 Bad Soden am Taunus(DE)**
Erfinder: **Usinger, Patricia, Dr.**
**Hauptstrasse 54**
**W-6239 Eppstein/Taunus(DE)**
Erfinder: **Siegel, Herbert, Dr.**
**Am Alten Birnbaum 10a**
**W-6238 Hofheim am Taunus(DE)**
Erfinder: **Alpermann, Hans Georg, Dr.**
**Am Eichkopf 10**
**W-6240 Königstein/Taunus(DE)**
Erfinder: **Gerhards, Hermann Josef, Dr.**
**Wacholderweg 4**
**W-6238 Hofheim am Taunus(DE)**

**EP 0 281 121 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft substituierte 1-(1-Aryl-2-hydroxy-ethyl)-imidazoleeinschließlich ihrer Salze, Verfahren zu ihrer Herstellung, diese Verbindungen enthaltende Arzneimittel und ihre Verwendung als Arzneimittel.

Es ist bereits vorgeschlagen worden (vgl. Deutsche Patentanmeldung P 36 28 545.5), substituierte Arylmethylazole und deren Salze, die aus der Umsetzung von 1-(Arylmethyl)-azolen mit Ketonen und Aldehyden hervorgehen, aufgrund ihrer psychopharmakologischen, insbesondere ihrer antidepressiven Wirkung zur Behandlung von depressiven Zuständen zu verwenden.

Der Erfindung lag die Aufgabe zu Grunde, Verbindungen zur Verfügung zu stellen, die bei guter Verträglichkeit eine therapeutisch verwertbare antidepressive Wirkung entfalten.

Die in der deutschen Patentanmeldung P 36 28 545.5 vorgeschlagenen Substanzen weisen ein oder zwei asymmetrische C-Atome auf. Es wurde nun gefunden, daß Verbindungen mit mindestens zwei asymmetrischen C-Atomen neben einer antimykotischen Wirkung insbesondere eine gute antidepressive Wirkung bei gleichzeitiger guter Verträglichkeit entfalten.

Die Erfindung ist daher gerichtet auf 1-(1-Aryl-2-hydroxy-ethyl)-imidazole der Formel I,

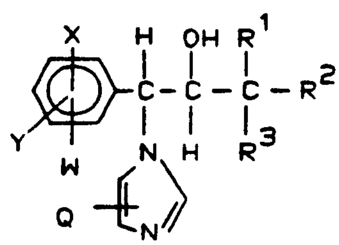

I

in der bedeuten

| | |
|---|---|
| X | H, $(C_1\text{-}C_4)$-Alkyl, Phenyl, F, Cl, Br, OH, $CF_3$, $(C_1\text{-}C_4)$ Alkoxy, $(C_1\text{-}C_4)$-Alkylthio, oder $-NR^4_2$ , in dem $R^4$ - gleich oder verschieden - $(C_1\text{-}C_4)$-Alkyl oder zusammen mit dem Stickstoffatom einen Pyrrolidin-, Piperidin- oder Morpholin-Rest bedeutet, |
| Y | H, $(C_1\text{-}C_4)$-Alkyl, F, Cl, Br, $(C_1\text{-}C_4)$-Alkoxy oder |
| X und Y | zusammen in 3,4-Stellung eine $-(CH_2)L$-Kette mit L = 3 oder 4, $-OCH_2CH_2-$, $-O\text{-}CH_2O-$ oder -CH = CH-CH = CH-, |
| W | H, $CH_3$ oder $OCH_3$, |
| $R^1$ | H, $(C_2\text{-}C_4)$-Alkyl, $(C_2\text{-}C_5)$-Alkenyl, $(C_2\text{-}C_5)$-Alkinyl, $(C_3\text{-}C_8)$-Cycloalkyl, $(C_4\text{-}C_8)$-Cycloalkenyl, $(C_3\text{-}C_7)$-Cycloalkyl-$CH_2$,$(C_4\text{-}C_7)$-1-Cycloalkenyl-$CH_2$, $(C_1\text{-}C_4)$-Alkoxymethyl, $(C_3\text{-}C_5)$-Alkenyloxymethyl oder 2-Propin-1-yloxymethyl, |

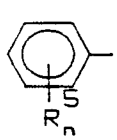
$-(C_1\text{-}C_3)$-Alkyl oder
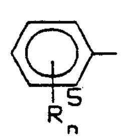
$CH{=}CH\text{-}CH_2$, worin

| | |
|---|---|
| $R^5$ | $CH_3$, $C_2H_5$, $OCH_3$, F, Cl, Br oder $CF_3$ und |
| n | 0,1 oder 2 bedeutet und für den Fall, daß $R^5$ $CF_3$ bedeutet, n 1 ist, |
| $R^2$ | $(C_1\text{-}C_4)$-Alkyl oder $(C_3\text{-}C_5)$-Alkenyl, |
| $R^3$ | $(C_1\text{-}C_4)$-Alkyl oder |
| $R^1$ und $R^3$ oder $R^2$ und $R^3$ | zusammen mit dem C-Atom, an dem sie gebunden sind, unsubstituiertes oder durch 1-3 $CH_3$ und/ oder $OCH_3$ und/oder C1 substituiertes $(C_3\text{-}C_7)$-Cyloalkyl, $C_7$-Bicycloalkyl, $(C_4\text{-}C_7)$-Cycloalkenyl oder $C_7$-Bicycloalkenyl, wobei in den Cycloalkenyl- und Bicycloalkenyl-Resten die C,C-Doppelbindung nicht in der 1-Position steht, oder $(Oxa\text{-}C_2\text{-}C_5)$-Cycloalkyl und |

| | |
|---|---|
| Q | H oder $CH_3$ oder $C_2H_5$ in 4- und/oder 5-Stellung sowie deren physiologisch verträgliche Säureadditionssalze und deren Stereoisomere und optisch aktiven Enantiomere. |

Bevorzugt sind Verbindungen der Formel I, in denen mindestens einer der Substituenten die folgende Bedeutung hat:

| | |
|---|---|
| X | H, ($C_1$-$C_4$)-Alkyl, Phenyl, F, Cl, Br, OH, ($C_1$-$C_4$)-Alkoxy, 3-$N(CH_3)_2$ oder 3-$CF_3$, |
| Y | H, $CH_3$, Cl oder $OCH_3$ oder |
| | X und Y zusammen in 3,4-stellung eine - $(CH_2)_4$-, -O-$CH_2$-O- oder -CH = CH-CH = CH- Kette, |
| W | H, |
| $R^1$ | ($C_2$-$C_4$)-Alkyl, ($C_2$-$C_5$)-Alkenyl, 2-Propin-1-yl, ($C_3$-$C_6$)-Cycloalkyl, ($C_1$-$C_4$)-Alkoxymethyl, ($C_3$-$C_5$)-Alkenyloxymethyl oder 2-Propin-1-yloxymethyl, |

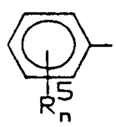 -($C_1$-$C_3$)-Alkyl, worin

| | |
|---|---|
| $R^5$ | $OCH_3$, F oder Cl und n 0 oder 1 bedeutet, |
| $R^2$ und $R^3$ | ($C_1$-$C_4$)-Alkyl oder |
| $R^1$ und $R^3$ oder $R^2$ und $R^3$ | zusammen mit dem C-Atom, an dem sie gebunden sind, unsubstituiertes oder durch 1 oder 2 $CH_3$ und/oder $OCH_3$ substituiertes ($C_3$-$C_7$)-Cycloalkyl, $C_7$-Bicycloalkyl oder $C_7$-Bicycloalkenyl, dessen C,C-Doppelbindung nicht in der 1-Position steht, oder (Oxa-$C_3$-$C_5$)-Cycloalkyl und |
| Q | H oder $CH_3$ in 4- oder 5-Stellung. |

Besonders bevorzugt sind diejenigen Verbindungen der Formel I, in denen mindestens einer der Substituenten die folgende Bedeutung hat:

| | |
|---|---|
| X | H, $CH_3$, Phenyl, F, Cl, Br, 3- oder 4-$OCH_3$ oder 3-$CF_3$, |
| Y, | H, $CH_3$, 3- oder 4-Cl oder 3- oder 4-$OCH_3$ oder |
| X und Y | zusammen in 3,4-Stellung eine -CH = CH-CH = CH-Kette, |
| W | H |
| $R^1$ | ($C_2$-$C_4$)-Alkyl, ($C_3$-$C_5$)-Alkenyl, 2-Propin-1-yl, Benzyl, ($C_1$-$C_4$)-Alkoxymethyl oder($C_3$-$C_4$)-Alkenyloxymethyl, |
| $R^2$ und $R^3$ | ($C_1$-$C_4$)-Alkyl oder |
| $R^1$ und $R^3$ oder $R^2$ und $R^3$ | zusammen mit dem C-Atom, an dem sie gebunden sind, ($C_3$-$C_7$)-Cycloalkyl, 2-Norbornyl, Norbornen-2-yl oder (Oxa-$C_3$-$C_5$)-Cycloalkyl und |
| Q | H oder $CH_3$ in 4- oder 5-Stellung. |

In diesem Zusammenhang ist unter dem Ausdruck "($C_1$-$C_3$)-,($C_1$-$C_4$)-, ($C_2$-$C_4$)-Alkyl" jeweils ein unverzweigter oder verzweigter Alkyl-Rest, unter dem Ausdruck "($C_2$-$C_5$)-,($C_3$-$C_4$)- oder ($C_3$-$C_5$)-Alkenyl" oder ($C_2$-$C_5$)-Alkinyl jeweils ein unverzweigter oder verzweigter Alkenyl- bzw. Alkinyl-Rest und unter dem Ausdruck "($C_1$-$C_4$)-Alkoxy oder ($C_1$-$C_4$)-Alkylthio" ein unverzweigter oder verzweigter Alkoxy- oder Alkylthio-Rest, unter dem Ausdruck ($C_3$-$C_8$)-Cycloalkyl und ($C_4$-$C_8$)-Cycloalkenyl jeweils ein gesättigter bzw. ungesättigter cyclischer Kohlenwasserstoffrest mit insgesamt 3-8 bzw. 4-8 Kohlenstoffatomen, wobei diese Kohlenstoffatome sowohl Glieder des Ringes wie auch ($C_1$-$C_3$)-Alkyl-Substituenten sein können, und unter dem Ausdruck $C_7$-Bicycloalkyl bzw. $C_7$-Bicycloalkenyl ein gesättigtes bzw. ungesättigtes, bicyclisches Ringsystem mit 7 Ring-Kohlenstoffatomen zu verstehen.

Bei den für "$R^1$ und $R^3$ oder $R^2$ und $R^3$ zusammen" genannten Bedeutungen ist unter den Ausdrücken ($C_3$-$C_7$)-Cycloalkyl bzw. ($C_4$-$C_7$)-Cycloalkenyl jeweils unter Einschluß des die Reste $R^1$ und $R^3$ bzw. $R^2$ und $R^3$ tragenden Kohlenstoffatoms ein aus 3-7 Kohlenstoffatomen bestehendes, gesättigtes Ringsystem bzw. aus 4-7 Kohlenstoffatomen bestehendes, ungesättigtes Ringsystem und unter den Ausdrücken $C_7$-Bicycloalkyl bzw. $C_7$-Bicycloalkenyl jeweils unter Einschluß des die Reste $R^1$ und $R^3$ oder $R^2$ und $R^3$ tragenden Kohlenstoffatoms ein gesättigtes bzw. ungesättigtes, bicyclisches, aus 7 Ringkohlenstoffatomen bestehendes Ringsystem zu verstehen, wobei im Fall von ungesättigten Ringsystemen die C,C-Doppelbindung in einer der möglichen Positionen steht.

Unter dem Ausdruck (Oxa-$C_2$-$C_5$)- bzw. (Oxa-$C_3$-$C_5$)-Cycloalkyl ist, unter Einschluß des die Reste $R^1$ und $R^3$ oder $R^2$ und $R^3$ tragenden C-Atoms, ein 3 bis 6 bzw. 4 bis 6 gliedriges, durch $R^1$ bzw. $R^2$ substituiertes Ringsystem zu verstehen, das aus 2 bis 5 bzw. 3-5 C-Atomen und einem Sauerstoffatom als weiterem Ringglied besteht. Als Beispiele hierfür seien das Oxetan, Tetrahydrofuran- und Tetrahydropyran-Gerüst genannt.

Die Verbindungen der Formel I enthalten mindestens zwei asymmetrische C-Atome und zwar diejenigen, woran der Imidazolrest und die Hydroxygruppe gebunden sind. Außerdem ist, falls $R^1$, $R^2$ und $R^3$ verschieden sind oder $R^1$ und $R^3$ oder $R^2$ und $R^3$ gemeinsam ein unsymmetrisches Ringsystem bilden, ein drittes asymmetrisches C-Atom vorhanden. Weitere asymmetrische C-Atome können in den Resten $R^1$, $R^2$ und/oder $R^3$ enthalten sein. Demzufolge fallen die Verbindungen I bei der Synthese mindestens als Racemate an. Je nach Anzahl der Asymmetriezentren und je nach dem Grad der Stereoselektivität unter den angewandten Bedingungen treten darüber hinaus Stereoisomere in Form von Racematen auf. Beispielsweise können bei der Synthese solcher Verbindungen der Formel I, die zwei Asymmetriezentren enthalten, zwei Diastereomeren-Racemate in gleichen oder unterschiedlichen Mengen gebildet werden. Häufig fallen solche Diastereomeren-Racemate infolge von Stereoselektivität in unterschiedlichen Mengen an, oder im Extremfall wird nur eines gebildet. Die Erfindung betrifft daher auch die möglichen Stereoisomeren, im einfachsten Fall die Diastereomeren, der Verbindungen I in Form ihrer Racemate oder in Form der optisch aktiven Enantiomeren, sowie deren pharmazeutisch abzeptablen Salze.

Die Verbindungen der Formel I können beispielsweise nach drei Verfahren hergestellt werden. Es ist bereits vorgeschlagen worden (vgl. Deutsche Patentanmeldung P 36 28 545.5), 1-Arylmethyl-azole nach Deprotonierung mit starken Basen an der Methylengruppe mit Carbonylverbindungen zu hydroxyalkylieren, wobei man mit stark solvatisierenden Lösungsmitteln und/oder mit zwei Äquivalenten einer solchen starken Base arbeitet. Aus Org. React. 26, S. 128 (1979) war andererseits bekannt, daß bei der Umsetzung von 1-Benzylimidazol mit n-Butyllithium in Diethylether bei Temperaturen zwischen 0 °C und -70 °C und anschließender Zugabe von Carbonylverbindungen nur Hydroxyalkylierung in der 2-Stellung des Imidazolrings stattfindet.

Es ist weiterhin vorgeschlagen worden (vgl. Deutsche Patentanmeldung P 36 28 545.5), 2-Aryl-2-(1-azolyl)-1-substituierte Ethanole herzustellen, indem man Oxirane der Formel IIa,

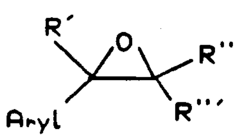

IIa

worin Aryl, R′, R″ und R‴ die in der Deutschen Patentanmeldung P 36 28 545.5 (dort R′ = $R^1$, R″ = $R^3$ und R‴ = $R^4$) genannten Bedeutungen besitzen, mit einer Verbindung der Formel IIIa,

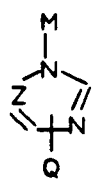

IIIa

in der M Wasserstoff oder ein Alkali- oder Erdalkalimetall bedeutet, und Q und Z die in der Deutschen Patentanmeldung P 36 28545.5 angegebene Bedeutung haben, bei
einer Temperatur zwischen -20 °C und 150 °C, bevorzugt in einem polaren Lösungsmittel nucleophil öffnet und dann weiter mit einer Protonensäure umsetzt.

Analog diesen in der Deutschen Patentanmeldung P 36 28 545,5 vogeschlagenen Herstellungsverfahren werden nun die Verbindungen der Formel I hergestellt, indem man

A) ein 1-Benzylimidazol der Formel IV,

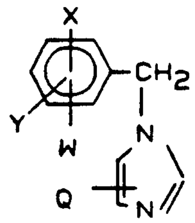

IV

worin X, Y, W und Q die oben angegebenen Bedeutungen haben, in einem aprotischen, zweckmäßig polaren Lösungsmittel bei einer Temperatur zwischen +40 °C, vorzugsweise 0 °C, und -100 °C, vorzugsweise -80 °C, mit einem oder bis zu zwei Äquivalenten einer starken Base versetzt und anschließend zuerst mit einem Aldehyd der Formel V,

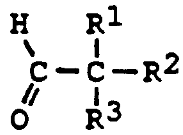

V

in der $R^1$, $R^2$ und $R^3$ die oben genannten Bedeutungen haben, und danach mit einer Protonensäure, vorzugsweise mit Wasser, umsetzt
oder
B) ein Oxiran der Formel II,

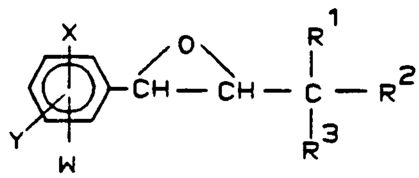

II.

in der X, Y, W, $R^1$, $R^2$ und $R^3$ die oben angegebenen Bedeutungen haben, bei einer Temperatur zwischen -20 °C, vorzugsweise 0 °C und 150 °C, vorzugsweise 60 °C, bevorzugt in einem polaren Lösungsmittel, durch Umsetzung mit einer Verbindung der Formel III,

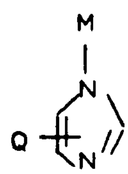

III

in der M Wasserstoff oder ein Alkali- oder Erdalkalimetall bedeutet und Q die oben angegebenen Bedeutung hat, nucleophil öffnet und dann mit einer Protonensäure versetzt,
oder
C) bei Verbindungen der Formel I, die nach Verfahren A) oder B) hergestellt worden sind, nach bekannten Methoden einen Substituenten X und/oder $R^1$ und/oder $R^2$ in einen anderen Substituent X und/oder $R^1$ und/oder $R^2$ umwandelt, und gegebenenfalls die nach den Verfahren A), B) und/oder C) erhaltenen Verbindungen der Formel I mit anorganischen oder organischen Säuren in ihre physiologisch verträglichen Säureadditionssalze überführt und gegebenenfalls eine nach A), B) oder C) erhaltene Verbindung in ihre Stereoisomeren und/oder ihre optisch aktiven Enantiomeren auftrennt.

Zur Bildung von Süureadditionssalzen kommen alle Säuren, die physiologisch verträgliche Salze bilden, in Frage. Dazu gehören sowohl anorganische Säuren als auch mono-, bi- und trifunktionelle organische Säuren, wie beispielsweise Chlor-, Brom- oder Jodwasserstoff-, Schwefel-, Phosphor-, Salpeter-,

Benzolsulfon-, Toluolsulfon-, Sulfamin-, Methylschwefel-, Essig-, Propion-, Öl-, Palmitin-, Stearin, Malon-, Malein-, Bernstein-, Glutar-, Äpfel-, Wein-, Zitronen-, Fumar-, Milch-, Glykol-, Brenztrauben-, Benzoe-, Toluyl-, Glutamin-, Furancarbon-, Salicyl- oder Mandelsäure. Bevorzugt werden Salze mit physiologisch verträglichen anorganischen Säuren, stark bis mittelstark sauren Derivaten solcher Säuren oder mit Bernsteinsäure L(+)-Weinsäure, D(-)-Weinsäure, Äpfelsäure, Fumarsäure, (S)-(+)- oder (R)-(-)-Mandelsäure.

Bei dem Verfahren A) kommen als starke Basen vor allem Alkalimetallhydride, wie z.B. Natriumhydrid, oder Erdalkali- und Alkalimetallalkyle, wie z.B. n-Butyllithium, tert.-Butyllithium, Methyllithium, Phenyllithium oder Methylmagnesiumchlorid oder -bromid oder metallierte Amine, wie z.B. Lithiumdiisopropylamid, Kalium- oder Natriumamid, in Frage, vorzugsweise werden Lithiumalkyle, wie n-Butyllithium oder Lithiumdiisopropylamid verwendet.

Die Anwendung von zwei Äquivalenten starker Basen ist bei der Mehrzahl der gemäß der Erfindung in den als Ausgangsstoff zu verwendenden 1-Benzylimidazolen vorkommenden Substituenten, wie auch bei im Benzylrest unsubstituierten solchen Verbindungen der Formel IV angebracht. Eine Ausnahme hiervon machen 1-Benzylimidazole, die im Phenylrest mit ausgesprochenen Akzeptor-Substituenten, wie z.B. $CF_3$, oder mit F, Cl und/oder Br in ortho- und/oder para-Postion substituiert sind. Bei diesen und bei 1-(3-Chlorphenyl-methyl)-imidazolen genügt die Anwendung von einem Äquivalent einer starken Base, vorzugsweise von Butyllithium, um ausreichend gute Ausbeuten an Verbindungen der Formel I zu erhalten.

Die Reaktion kann in den für metallorganische Reaktionen gebräuchlichen Lösungsmittel, wie z.B. Dimethylformamid, Dimethylsulfoxid, Dimethoxyethan, Diethylether oder vorzugsweise Tetrahydrofuran durchgeführt werden. Es können auch Mischungen verschiedener aprotischer Lösungsmittel, darunter auch Mischungen von polaren und unpolaren Lösungsmitteln, angewendet werden.
Außerdem können als Lösungsmittel Zusätze wie N, N, N′, N′-Tetramethylethylendiamin oder Hexamethylphosphorsäuretriamid Verwendung finden.

Die erfindungsgemäße Umsetzung mit der starken Base und dem Aldehyd der Formel V kann bei Temperaturen zwischen +40 °C und -100 °C erfolgen, geschieht jedoch vorzugsweise im Bereich zwischen 0 °C und -80 °C. Die Umsetzung kann auch bei einer Temperatur oberhalb von +40 °C ausgeführt werden. Die Verbindungen der Formel I werden in der bei metallorganischen Reaktionen üblichen und bekannten Weise isoliert. Die Reinigung der Verbindungen der Formel I erfolgt in der Regel durch Umkristallisation aus einem organischen Lösungsmittel, wie z.B. Hexan, Cyclohexan, Ethanol, Essigsäureethylester, Diisopropylether oder Acetonitril oder einem Lösungsmittelgemisch oder durch Säulenchromatographie an Kieselgel.

Die als Ausgangsstoff verwendeten, unsubstituierten oder erfindungsgemäß substituierten 1-Benzylimidazole der Formel IV werden nach bekannten Methoden durch Alkylierung von Imidazol bzw. in 3- und/oder 4-Stellung substituierten Imidazolen mit Benzylhalogeniden bzw. mit 2-Chlor- oder Brommethylnaphthalin erhalten.

Die als Ausgangsstoffe benötigten Benzylhalogenide bzw. Chlor- oder Brommethyl-naphthaline sind großteils bekannt oder können nach bekannten Methoden hergestellt werden, beispielsweise durch Umsetzung entsprechender Hydroxymethyl-Verbindungen mit Thionylchlorid oder durch Bromierung entsprechender Methyl-Verbindungen mit NBS (N-Bromsuccinimid).

Aldehyde der Formel V sind teils bekannt oder können nach bekannten Methoden hergestellt werden. Beispielsweise können solche Aldehyde mit tertiär ständiger Formylgruppe durch Substitution von Aldehyden der Formel VI,

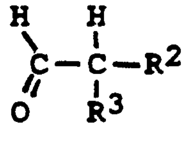

VI

wobei $R^2$ und $R^3$ die oben angegebenen Bedeutungen haben, in α-Stellung mit geeigneten Alkyl- bzw. Alkenyl- oder Alkinylierungs-Reagenzien der Formel $E\text{-}R^1$, in der $R^1$ die oben angegebenen Bedeutungen hat und E, Cl, Br, J oder eine der üblicherweise für solche Zwecke verwendeten Abgangsgruppen, wie z.B. Methansulfonyloxy, Benzol- oder Toluolsulfonyloxy, bedeutet, aufgebaut werden. Derartige Reaktionen können vorteilhaft unter den Bedingungen einer Phasentransferreaktion ausgeführt werden, indem man die Reaktionspartner in einem geeigneten Lösungsmittel, wie z.B. Ether, Dioxan, Tetrahydrofuran, Methylenchlorid, tert.-Butanol, einem aliphatischen, cycloalphatischen oder aromatischen Kohlenwasserstoff wie Hexan, Cyclohexan, Benzol, Toluol oder Xylol, Anisol oder Chlorbenzol unter kräftigem Rühren in Gegenwart eines Phasentransferkatalysators und entweder einem gepulverten Alkalihydroxid, wie z.B. Natrium- oder Kaliumhydroxid oder einer konzentrierten wäßrigen Lösung derselben, vorzugsweise in einem Temperaturbereich von 20 °C bis 120 °C aufeinander einwirken läßt.

Geeignete Phasentransferkatalysatoren sind z.B. Trialkylbenzylammonium- oder Tetraalkylammoniumhalogenide, - hydroxide, -hydrogensulfate mit vorzugsweise 1 bis 12 C-Atomen im Alkylrest oder Kronenether, wie z.B. 12-Crown-4, 15-Crown-5, 18-Crown-6 oder Dibenzo-18-crown-6.

Andererseits können Aldehyde der Formel V auch aufgebaut werden, indem man z.B. Aldehyde der Struktur VII

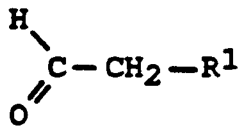

VII

auf prinzipiell ähnliche Weise wie vorstehend beschrieben in α-Stellung einfach oder zweifach alkyliert oder alkenyliert.

Ein anderer Weg zur Herstellung von Aldehyden der Formel V besteht darin, daß man mit literaturbekannten Methoden, z.B. mit Pyridiniumchlorochromat (PCC), entsprechende Alkohole oxidiert. Diese Methode wird u.a. beispielsweise zur Herstellung der Oxacycloalkan-aldehyde aus entsprechenden Hydroxymethyl-Verbindungen angewandt.

Beim Verfahren B) werden bevorzugt Verbindungen der Formel III verwendet, worin M, Li, Na oder K, insbesondere Na, und Q Wasserstoff oder $CH_3$ bedeutet. Die Verbindungen der Formel III sind bekannt oder können nach bekannten Methoden hergestellt werden.

Oxirane der Formel II erhält man nach literaturbekannten Methoden. Die Herstellung der zugrundeliegenden Olefine erfolgt ebenfalls nach bekannten Methoden. Die Oxidation der Olefine zum Epoxid geschieht mit einer organischen Persäure, wie z.B. Peressigsäure oder m-Chlorperbenzoesäure nach literaturbekannten Methoden.

Bei dem Verfahren C) wird bei Verbindungen der Formel I, die nach Verfahren A) oder B) hergestellt worden sind, nach bekannten Methoden ein Substituent X und/oder ein Substituent $R^1$ und/oder ein Substituent $R^2$ in einen anderen Substituent X und/oder einen anderen Substituent $R^1$ und/oder einen anderen Substituent $R^2$ ungewandelt.

Beispielsweise wird nach diesem Verfahren C) ein Benzyloxy-Rest in der Phenylgruppe durch katalytische Hydrogenolyse in eine Hydroxygruppe und Toluol übergeführt, oder ein Rest $R^1$ und/oder ein Rest $R^2$, die jeweils ($C_2$-$C_5$)-oder ($C_3$-$C_5$)-Alkenyl bedeuten, wird durch katalytische Hydrierung in einen ($C_2$-$C_5$)- bzw. ($C_3$-$C_5$)-Alkyl-Rest übergeführt.

Als Katalysatoren sind hierzu im ersten Fall z.B. verschiedene Arten von feinverteiltem Palladium auf Aktivkohle oder auf Calziumcarbonat oder auf Bariumsulfat und im zweiten Fall, der Hydrierung eines Alkenyl-Restes, verschiedenen Arten von feinverteiltem Palladium, wie vorstehend für den ersten Fall erwähnt, oder von feinverteiltem Platin auf Aktivkohle oder auf Aluminiumoxid oder Platinoxid geeignet.

Die Reaktionszeiten betragen je nach Verfahrensvariante und je nach Temperaturbereich wenige Minuten bis einige Stunden.

Die erfindungsgemäßen Verbindungen der Formel I enthalten zwei oder mehrere Asymmetriezentren. Demzufolge erhält man bei der Synthese in der Regel Stereoisomerengemische. Die einzelnen Stereoisomeren können dabei infolge unterschiedlicher Stereoselektivität in unterschiedlichen Mengen anfallen. Bei hoher Stereoselektivität beispielsweise kann im Fall von Diastereomeren überwiegend oder nahezu ausschließlich nur ein Diastereomeres, als Racemat, entstehen. Diastereomere Racemate wie auch Diastereomere, bei denen beispielsweise ein Asymmetriezentrum in einheitlicher (R- oder S-) Konfiguration vorliegt, von Verbindungen der Formel I können in üblicher Weise, z.B. durch selektive, fraktionierte Kristallisation oder Säulenchromatographie getrennt werden.

Die diastereomeren Racemate können ihrerseits in üblicher Weise in ihre optischen Antipoden (Enantiomeren) getrennt werden.

Die Trennung von Racematen und diastereomeren Racematen kann nach im Prinzip bekannten Methoden erfolgen, beispielsweise durch fraktionierte Kristallisation diastereomerer Salze mit optisch aktiven Carbonsäuren oder Sulfonsäuren einheitlicher Konfiguration oder durch Chromatographie an chiralen Trennmedien (Trägerstoffen). Als optisch aktive, chirale Säuren sind beispielsweise geeignet: L(+)- oder D(-)-Weinsäure, D(+)- oder L(-)-Äpfelsäure, R-(-)- oder (S)-(+)-Mandelsäure, L(+)-Milchsäure, (+)-Campher-10-sulfonsäure oder (+)-3-Bromcampher-8-sulfonsäure. Diastereomere Salze derartiger chiraler Säuren mit Verbindungen der Formel I werden aus geeigneten Lösungsmitteln oder Lösungsmittelgemischen fraktioniert umkristallisiert, bis ein konstanter Drehwert erreicht ist.

Durch Einwirkung einer äquivalenten Menge oder eines Überschusses Base können die optisch reinen diastereomeren Salze in die reinen Enantiomeren der Verbindungen I übergeführt und als solche isoliert werden.

Werden bei der Synthese von Verbindungen I nach Verfahren A) Aldehyde der Formel V in Form optisch reiner Enantiomerer verwendet, dann können bei ausreichender Kristallisationstendenz mitunter die gebildeten Diastereomeren ohne chiralen Hilfsstoff, z.B. einer chiralen Säure, in bekannter Weise durch fraktionierte Kristallisation und/oder mittels chromatographischer Methoden in die optischen Antipoden getrennt werden.

Die chromatographische Trennung von Diastereomeren oder Racematen an chiralen Trägerstoffen ist mittels konventioneller Säulenchromatographie möglich, eine effektivere Trennung wird in der Regel mit der Mitteldruck-oder Hochleistungsflüssigkeitschromatographie erreicht.

Die Verbindungen der Formel I und ihre Säureadditionssalze sind wertvolle Arzneimittel. Sie zeichnen sich durch eine starke psychotrope, insbesondere antidepressive Wirkung aus und können daher zur Behandlung von depressiven Zuständen verwendet werden. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Säugers. Bei oraler Dosierung werden befriedigende Ergebnisse mit Dosen von ab 0,1 mg, vorzugsweise ab 0,4 mg, bis zu 100 mg, vorzugsweise bis zu 20 mg, einer Verbindung der Formel I pro kg Körpergewicht erreicht.

Die Verbindungen der Formel (I) und ihre Säureadditionssalze stellen wertvolle Psychopharmaka dar. Sie sind in den für Antidepressiva spezifischen Testmodellen biochemischer und pharmakologischer Art sehr gut wirksam.

Die erfindungsgemäßen Verbindungen antagonisieren mit einer $ED_{50}$ von 0,5 - 60 mg/kg oral appliziert, die Tetrabenazin-Ptosis an der Maus und an der Ratte. Sechs männl. Tieren wird die entsprechende Menge eines Homogenats der jeweiligen Substanz in wäßriger Carboxymethylzellulose eine Stunde vor der Behandlung mit Tetrabenazin (40 mg/kg intraperitoneal) mit Schlundsonde gefüttert. Die Tetrabenazin-Ptosis wird durch diese Vorbehandlung antagonisiert.

Die Verbindungen I und ihre Säureadditionssalze potenzieren die Toxizität von Yohimbin an Mäusen. Antidepressiva des Typs I mit 0,5 bis 100 mg/kg per os verursachen die Erhöhung der Todesrate einer sonst nicht tödlichen Dosis von Yohimbinhydrochlorid (20 mg/kg subkutan gegeben).

Ein wesentlicher Befund ist, daß die Verbindungen I und ihre Salze an Ratten und Mäusen nach Dosierungen in einem therapeutisch relevanten Bereich keine Stereotypien verursachen.

Die antidepressive Wirkung und die Brauchbarkeit zur Behandlung von depressiven Zuständen wurde weiterhin anhand der Hemmung der Wiederaufnahme von Noradrenalin an Mäusehirn-Synaptosomen-Präparationen demonstriert.

Die Verbindungen sowie ihre Säureadditionssalze sind besonders wertvoll durch eine von den bisher bekannten Antidepressiva abweichende Struktur. Bekannten Handelsprodukten sind sie in ihrer Wirkung bei geringerer Toxizität gleichwertig oder überlegen.

Die Verbindungen der Formel I sind außerdem antimykotisch wirksam. Sie wirken in vitro sehr gut gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum und außerdem gegen Protozoen wie Trichomonas vaginalis oder T. fetus, und auch gegen gram-positive und gramnegative Bakertien.

Ebenso besteht ein sehr guter Effekt gegen verschiedene Erreger der Hautmykosen (z.B. Trichophyton mentagrophytes) am Meerschweinchen, insbesondere nach lokaler Anwendung.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutischen geeigneten Trägerstoffen sind feste, halbfeste oder flüssige Verdünnungsmittel, Füllstoffe und Formulierungshilfsmittel jeder Art zu verstehen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions oder Sprays in Betracht.

Die therapeutisch wirksamen Verbindungen sollen in den oben aufgeführten pharmazeutischen Zubereitungen zweckmäßig in einer Konzentration von etwa 0,01, vorzugsweise von 0,10 bis 99,0 vorzugsweise bis 50,0, Gewichtsprozenten der Gesamtmischung vorhanden sein.

Die Anwendungskonzentrationen für Lösungen, Gelees, Cremes oder Salben sowie Aerosole in Form von Spray betragen im allgemeinen zwischen 0,1 - 20, vorzugsweise 0,5 - 5 Gewichstprozenten.

Für die lokale Anwendung als Antimykotika können z.B. Suspensionen, Lösungen Gelees, Cremes, Salben oder Suppositorien verwendet werden.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und im Fall von antimykotisch wirksamen Verbindungen in der Human- und Veterinärmedizin zur Verhütung, Besserung und/oder Heilung der oben angeführten Erkrankungen.

Bei antimykotisch wirkenden erfindungsgemäßen Verbindungen hat es sich sowohl in der Human- als auch in der Veterinärmedizin als vorteilhaft erwiesen, den oder die erfindungsgemäßen Wirkstoffe in Gesamtmengen von etwa 0,05 bis 200, vorzugsweise 0,1 bis 100, insbesondere 0,5 bis 30 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen verabreicht.

Die Wirkstoffe oder die pharmazeutischen Zubereitungen können lokal, parenteral, intraperitoneal und/oder rectal appliziert werden.

Die als Antidepressiva verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht pharmakologisch wertvolle Stoffe enthalten können, werden z.B. mittels konventioneller Misch-Granulier- und Dragierverfahren, hergestellt und enthalten 0,1 % bis etwa 75 % bevorzugt etwa 1 % bis etwa 50 % des Wirkstoffs.

Die orale Anwendung erfolgt in pharmazeutisch üblichen Zubereitungen, beispielsweise in Form von Tabletten, Dragees oder Kapseln, die z.B. pro Tagesdosis 5, vorzugsweise 30, bis 600 mg, vorzugsweise bis 300 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowei dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen der Formel I sind auch fungizid wirksam. Das Wirkungsspektrum der beanspruchten Verbindungen erfaßt eine Vielzahl verschiedener phytopathogener Pilze, wie z.B. Piricularia oryzae, Pellicularia sasakii, verschiedene Rostarten, Cercospora-Arten und echte Mehltaupilze im Obst-, Gemüse-, Getreide- und Zierpflanzenbau.

Die folgenden Beispiele erläutern die Herstellung der Verbindungen. Die in den Beispielen beschriebenen Umsetzungen wurden unter einer Stickstoffatmosphäre durchgeführt.

Beispiel 1

1-(1-Allyl-1-cyclohexyl)-2-(3-chlorphenyl)-2-(1-imidazolyl)-ethanol

Zu einer Lösung von 3,85 (20 mmol) 1-(3-Chlorbenzyl)-imidazol in 40 ml abs. Tetrahydrofuran (THF) wurden bei -70 °C 13,6 ml (20,1 mmol) einer 1,48 molaren Lösung von n-Butyllithium in Hexan getropft. Man rührte 30 Min. bei -70 °C nach und tropfte dann bei -70 °C in ca. 20 Min. eine Lösung von 3,60 g 85 %igem (20 mmol) 1-Allyl-1-formylcyclohexan in 30 ml abs. Tetrahydrofuran (THF) zu. Anschließend rührte man 15 Min. bei ca. - 70 °C nach, ließ die Reaktionsmischung in ca. 2 Stunden auf Zimmertemperatur aufwärmen, versetzte unter Kühlung bei ca. 5-16 °C mit 200 ml Wasser, rührte die Mischung 45 Min. bei ca. 5-15 °C nach, trennte die Phasen und extrahierte die wässrige Phase mehrmals mit Ether. Die vereinigten organischen Lösungen wurden mit ca. 20 ml Waser ausgewaschen, getrocknet, filtriert und im Vakuum eingedampft. Den Extraktrückstand (6,8 g) löste man in Acetonitril, wonach kristallines 1-(1-Allyl-1-cyclohexyl)-2-(3-chlorphenyl)-2-(1-imidazolyl)-ethanol (2,6 g) ausfiel. Das Produkt (2,6 g) wurde aus 20 ml Acetonitril umkristallisiert. Danach fielen 2,3 g reines Produkt (Fp. 119 °C) an. Aus den Mutterlaugen wurden nach dem Einengen derselben weitere 0,6 g Produkt erhalten, das nach Umkristallisieren aus Acetonitril weitere 0,42 g reines Produkt (Fp. 119 °C) ergab. Die Ausbeute an reinem 1-(1-Allyl-1-cyclohexyl)-2-(3-chlorphenyl)-2-(1-imidazolyl)-ethanol betrug 2,72 g (≙ 39,4 % d. Th.).

| $C_{20}H_{25}ClN_2O$ (344,88) | | | | |
|---|---|---|---|---|
| berechnet: | C 69,65 | H 7,31 | Cl 10,28 | N 8,12 % |
| gefunden: | C 69,7 | H 7,3 | Cl 10,5 | N 8,4 % |

Beispiel 2

1-(3-Chlorphenyl)-3,3-dimethyl-1-(1-imidazolyl)-5-hexen-2-ol

Zu einer Lösung von 4,82 g (25 mmol) 1-(3-Chlorbenzyl)-imidazol in 50 ml abs. THF wurden bei -70 °C 16,2 ml (25,1 mmol) einer 1,55 molaren Lösung von n-Butyllithium in Hexan getropft. Man rührte 30 Min. bei -70 °C nach und tropfte dann bei ca. -70 °C in ca. 15 Min. eine Lösung von 3,75 g 75 %igem (25 mmol) (15 % Toluol enthaltenden) 2,2-Dimethyl-4-penten-aldehyd in 38 ml abs. THF zu. Anschließend rührte man 15. Min bei ca. -70 °C nach, ließ in ca. 2 Stunden auf Zimmertemperatur aufwärmen, versetzte unter Kühlung bei 1-10 °C mit 350 ml Wasser, rührte die Mischung 30 Min. bei 10-20 °C nach, trennte die Phasen und extrahierte die wäßrige Phase mehrmals mit Ether. Die vereinigten organischen Lösungen wurden wie im Beispiel 1 beschrieben weiter aufgearbeitet. Den Extraktrückstand (7,2 g) chromatographierte man unter Elution mit $CH_2Cl_2$/Hexan 1:2- und 1:1-Mischung, $CH_2Cl_2$ und $CH_2Cl_2/C_2H_5OH$-Mischungen mit steigendem $C_2H_5OH$-Gehalt (bis max. 2 Vol.-%) an einer Kieselgel S/$CH_2Cl_2$ : Hexan 1:2-Säule (Ø 2,8 cm, Höhe 43 cm).

Nach Elution von 0,60 g Vorfraktionen wurden 5,0 g Produkt eluiert, in dem die gesuchte Verbindung stark angereichert war. Aus diesen Anteil wurden durch Kristallisation aus Diisopropylether 3,10 g (≙ 40,7 % Ausbeute) reines 1-(3-Chlorphenyl)-3,3-dimethyl-1-(1-imidazolyl)-5-hexen-2-ol vom Fp. 95 °C erhalten.

| $C_{17}H_{21}ClN_2O$ (304,83) | | | | |
|---|---|---|---|---|
| berechnet: | C 66,98 | H 6,94 | Cl 11,63 | N 9,19 % |
| gefunden : | C 67,4 | H 7,1 | Cl 12,0 | N 9,2 % |

Beispiel 3

1-(3-Chlorphenyl)-3,3-dimethyl-1-(4(5)-methyl-1-imidazolyl)-5-hexen-2-ol

Zu einer Lösung von 5,17 g (25 mmol) 1-(3-Chlorbenzyl)-4(5)-methyl-imdazol (6:4-Mischung) in 50 ml abs. THF wurden nacheinander, wie im Beispiel 2 beschrieben, bei -70 °C 16,2 ml 1,55 molare n-Butyllithium/Hexan-Lösung und eine Lösung von 3,75 g 75 %igem, 2,2-Dimethyl-4-pentenaldehyd in 38 ml

abs. THF getropft. Die weitere Bearbeitung erfolgte ebenso wie im Beispiel 2 beschrieben. Den Extraktrückstand (8,0 g) chromatographierte man durch Elution mit $CH_2Cl_2$ und $CH_2Cl_2/C_2H_5OH$-Mischungen mit steigendem $C_2H_5OH$-Gehalt (bis max. 2 Vol.-%) an einer Kieselgel S/$CH_2Cl_2$-Säule (∅ 2,8 cm, Höhe 46 cm). Nach Elution von 0,63 g Vorfraktionen wurden 0,75 g unreines Produkt eluiert, aus dem aus Diisopropylether 0,12 g 1-(3-Chlorphenyl)-3,3-dimethl-1-(4(5)-methyl-1-imidazolyl)-5-hexen-2-ol (als 4- und 5-Methyl-imidazolyl-Mischung) kristallin erhalten wurden. Danach wurden 4,80 g stark angerecihertes Produkt eluiert, aus dem aus Diisopropylether 3,45 g kristallines Produkt erhalten wurden. Insgesamt erhielt man 3,57 g (≙ 44,8 % Ausbeute) reines (als 4- und 5-Methyl-Mischung) 1-(3-Chlorphenyl)-3,3-dimethyl-1-(4(5)-methyl-1-imidazolyl)-5-hexen-2-ol vom Fp. 92° C.

| $C_{18}H_{23}ClN_2O$ (318,86) | | | | |
|---|---|---|---|---|
| berechnet: | C 67,80 | H 7,27 | Cl 11,12 | N 8,79 % |
| gefunden: | C 68,1 | H 7,4 | Cl 11,3 | N 8,5 % |

Beispiel 4

1-(2-Allyl-5-norbornen-2-yl)-2-(3-chlorphenyl)-2-(1-imidazolyl)-ethanol

Zu einer Lösung von 3,85 g (20 mmol) 1-(3-Chlorbenzyl)-imidazol in 40 ml abs. THF wurden bei -70 °C 13,8 ml (20 mmol) einer 1,45 molaren Lösung von n-Butyllithium in Hexan getropft. Nach 30 Min. Rühren bei -70 °C tropfte man bei ca. -70 °C in ca. 12 Min. eine Lösung von 4,39 g 74 %igem (20 mmol) 2-Allyl-5-norbornen -2-aldehyd in 30 ml abs. THF zu. Anschließend wurde im Beispiel 2 beschrieben weitergearbeitet. Der aus dem organischen Anteil nach Abdestillierten der Lösungsmittel verbleibende Rückstand (9,0 g) wurde im Diisopropylether-Hexan 2:2 gelöst, woraufhin kristallines Produkt ausfiel. Dieses wurde nach Isolierung mit 20 ml Acetonitril kurz aufgekocht, nach Kühlen der Mischung abgesaugt und mit Acetonitril gewaschen. Man erhielt 2,70 g (≙ 38 % Ausbeute) reines 1-(2-Allyl-5-norbornen-2-yl)-2-(3-chlorphenyl)-2-(1-imidazolyl)-ethanol in Form einer aus 2 Diastereomeren bestehenden Mischung vom Fp. 134-135 °C.

| $C_{21}H_{23}ClN_2O$ (354,87) | | | | |
|---|---|---|---|---|
| berechnet: | C 71,08 | H 6,53 | Cl 9,99 | N 7,89 % |
| gefunden : | C 71,2 | H 6,7 | Cl 10,3 | N 7,9 % |

Beispiel 5

1-(4-Diphenyl)-3,3-dimethyl-1-(1-imidazolyl)-4-phenyl-2-butanol

5,86 g (25 mmol) 1-(4-Diphenylmethyl)-imidazol in 50 ml abs. THF wurden bei -70 °C wie im Beispiel 2 beschrieben mit 16,2 ml 1,55 molarer n-Butyllithium/Hexan-Lösung metalliert. Bei -70 °C tropfte man dann eine Lösung von 4,51 g 90 %igem 2,2-Dimethyl-3-phenyl-propionaldehyd (25 mmol) in 35 ml abs. THF zu, rührte 20 Min. bei -70 °C, ließ in 2,5 Stunden auf Zimmertemperatur anwärmen, versetzte bei ca. 0 °C tropfenweise mit 250 ml Wasser und rührte 1 Stunde unter Eiskühlung nach. Dabei schied sich kristalline Substanz ab, die abgesaugt, mit Wasser und Ether gewaschen und danach in 100 ml $CH_2Cl_2/CH_3OH$ 1:1 gelßst wurde. Nach dem Filtrieren dampfte man diese Lösung im Vakuum ein und kochte den kristallinen Rückstand mit 35 ml Acetonitril 30 Min. auf. Nach Abkühlung auf 5-8 °C saugte man den Feststoff ab und wusch mit Acetonitril nach. Man erhielt 2,70 g (≙ 27,3 % Ausbeute) reines 1-(4-Diphenyl)-3,3-dimethyl-1-(1-imidazolyl)-4-phenyl-2-butanol vom Fp. 220 °C.

| $C_{27}H_{28}N_2O$ (396,54) | | | |
|---|---|---|---|
| berechnet: | C 81,78 | H 7,12 | N 7,07 % |
| gefunden : | C 81,6 | H 7,1 | N 6,9 % |

Beispiel 6

1-(3-Bromphenyl)-3,3-dimethyl-1-(1-imidazolyl)-5-hexen-2-ol

In 40 ml abs. Tetrahydrofuran (THF) wurde bei -30 ° C ausgehend von 2,06 g (20,4 mmol) Diisopropylamin und 13,4 ml 1,5 molarer n-Butyllithium/Hexan-Lösung (20 mmol) eine Lösung von Lithiumdiisopropylamid hergestellt. Hierzu tropfte man bei -70 ° C bis -78 ° C eine Lösung von 4,75 g (20 mmol) 1-(3-Brombenzyl)-imidazol in 25 ml abs. THF, rührte 30 Min. bei ca. -70 ° C nach, tropfte anschließend bei ca. -70 ° C eine Lösung von 3,22 g 70 %igem (20 mmol) 2,2-Dimethyl-4-penten-aldehyd in 30 ml abs. THF zu, rührte 20 Min. bei ca. -70 ° C nach und ließ in ca. 2,5 Stunden auf Zimmertemperatur aufwärmen. Anschließend wurde bei 0 ° C - 5 ° C mit 300 ml Wasser tropfenweise versetzt und 1 Stunde unter Eiskühlung gerührt. Nach der Trennung der Phasen extrahierte man die wäßrige Lösung mehrmals mit Ether. Die vereinigten organischen Lösungen wurden wie im Beispiel 1 beschrieben weiter aufgearbeitet. Den Extraktrückstand (7,3 g) chromatographierte man durch Elution mit $CH_2Cl_2$ und $CH_2Cl_2/C_2H_5OH$-Mischungen mit steigerdem $C_2H_5OH$-Gehalt (bis max. 2 Vol.-%) an einer Kieselgel S/$CH_2Cl_2$-Säule ($\phi$ 2,8 cm, Höhe 48 cm). Nach Elution von 2,0 g Vorfraktionen wurden 5,2 g Produkt, in dem die gesuchte Verbindung hoch angereichert war, eluiert. Aus diesen 5,2 g wurden durch Kristallisation aus Diisopropylether/Hexan 2:1 3,05 g (≙ 43,6 % Ausbeute) reines 1-(3-Bromphenyl)-3,3-dimethyl-1-(1-imidazolyl)-5-hexen-2-ol vom Fp. 126 ° C erhalten.

| $C_{17}H_{21}BrN_2O$ (349,29) | | | | |
|---|---|---|---|---|
| berechnet: | C 58,46 | H 6,06 | Br 22,88 | N 8,02 % |
| gefunden : | C 58,4 | H 6,1 | Br 23,8 | N 8,0 % |

Analog zu den Beispielen 1 bis 6 wurden folgende Verbindungen der Formel I erhalten:

Tabelle 1:

| Bsp. | X | Y | W | Q | $R^1$ | $R^2$ | $R^3$ | Fp. [°C] |
|---|---|---|---|---|---|---|---|---|
| 7 | 3-Cl | H | H | H | $CH_2-\underset{CH_3}{C}=CH_2$ | $CH_3$ | $CH_3$ | 102 |
| 8 | 3-Cl | 4-Cl | H | H | $CH_2-CH=CH_2$ | $-(CH_2)_5-$ | | 143 |
| 9 | 3-Cl | H | H | H | $CH_2\underset{CH_3}{C}=CH_2$ | $C_2H_5$ | $C_2H_5$ | ---(Öl) |
| 10 | 3-Cl | H | H | H | $CH_2$- 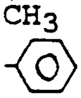 | $CH_3$ | $CH_3$ | 151 |
| 11 | 3-Cl | H | H | H | $CH_2$- 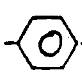 -Cl | $CH_3$ | $CH_3$ | 194 |
| 12 | 3-Cl | H | H | H | $CH_2$- 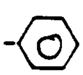 | $-(CH_2)_5-$ | | 188 |
| 13 | 4-$C_6H_5$ | H | H | H | $CH_2-CH=CH_2$ | $CH_3$ | $CH_3$ | 120 |
| 14 | 4-$C_6H_5$ | H | H | H | $CH_2-\underset{CH_3}{C}=CH_2$ | $CH_3$ | $CH_3$ | 140 |

Beispiel 15

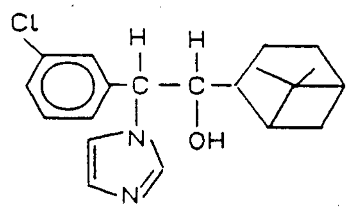

1-(6,6-Dimethyl-bicyclo[3,1,1]hept-2-yl)-2-(3-chlorphenyl)-2-(1-imidazolyl)-ethanol

7,70 g (40 mmol) 1-(3-Chlorbenzyl)-imidazol in 80 ml abs. THF wurden bei -70 °C wie im Beispiel 2 beschrieben mit 27 ml 1,48 molarer n-Butyllithium/Hexan-Lösung metalliert. Bei -70 °C tropfte man dann eine Lösung von 6,10 g (40 mmol) 2-Formyl-6,6-dimethyl-bicyclo[3,1,1]heptan(Myrtanal) in 60 ml abs. THF zu, rührte 20 Min. bei -70 °C und ließ in 2,5 Stunden auf Zimmertemperatur anwärmen. Die weitere Bearbeitung erfolgte analog wie im Beispiel 2 beschrieben. Der nach Eindampfen der vereinigten organischen Lösungen (Extrakte) verbleibende Rückstand (13,60 g) wurde durch Elution mit $CH_2Cl_2$ und $CH_2Cl_2/C_2H_5OH$-Mischungen mit steigendem $C_2H_5OH$-Gehalt (bis max. 3 Vol.-%) an einer Kieselgel S/$CH_2Cl_2$-Säule ($\phi$ 2,1 cm, Höhe 62,5 cm) chromatographiert. Nach Elution von 4,7 g Vorfraktionen und 2,4 g durch Nebenprodukte verunreinigtem Produkt wurden 6,4g Produkt eluiert, in dem die gesuchte Verbindung in Form von Stereoisomeren praktisch rein vorlag. Aus dieser Fraktion erhielt man durch Kristallisation aus Diisopropylether 1,52 g eines laut DC und $^1$H-NMR-Spektrum reinen Stereoisomeren des 1-(6,6-Dimethyl-bicyclo[3,1,1]hept-2-yl)-2-(3-chlorphenyl)-2-(1-imidazolyl)-ethanols vom Fp. 149-150 °C.

Die Ausbeute betrug 4,88 g (≙ 35,4 % d. Th.) an Stereoisomerenmischung und 1,52 g (≙ 11 % d. Th.) eines reinen, kristallinen Stereoisomeren.

| $C_{20}H_{25}ClN_2O$ (344,88) | | | | |
|---|---|---|---|---|
| berechnet: | C 69,65 | H 7,31 | Cl 10,28 | N 8,12 % |
| gefunden : | C 69,6 | H 7,2 | Cl 10,6 | N 8,3 % (Stereoisomerenmischung) |
| gefunden : | C 70,0 | H 7,0 | | N 8,0 % (reines Diastereomeres) |

Beispiel 16

3,3-Dimethyl-1-(1-imidazolyl)-1-phenyl-5-hexen-2-ol

Zu einer Lösung von 4,75 g (30 mmol) 1-Benzylimidazol und 3,49 g (30 mmol) N,N,N',N'- Tetramethylethylendiamin (TMEDA) in 60 ml abs. THF wurden bei -70 °C 40 ml 1,5 molare n-Butyllithium/Hexan-Lösung getropft. Man rührte 30 Min. bei -70 °C - -78 °C nach, tropfte dann bei ca. -70 °C in 25 Min. eine Lösung von 3,74 g 90 %igem (30 mmol) 2,2-Dimethyl-4-penten-aldehyd in 38 ml abs. THF zu, rührte 20 Min. bei ca. -70 °C nach und ließ in ca. 2 Stunden auf Zimmertemperatur anwärmen. Danach wurde bei 0 ° - 8 °C mit 350 ml Wasser versetzt, 30 Min. unter Eisbad-Kühlung nachgerührt und anschließend wurden die Phasen getrennt und die wäßrige Lösung mehrmals mit Ether extrahiert. Die vereinigten organischen Lösungen arbeitete man wie im Beispiel 1 beschrieben weiter auf. Der Extraktrückstand (8,4 g) wurde unter Elution mit $CH_2Cl_2$ und $CH_2Cl_2/C_2H_5OH$-Mischungen mit steigendem $C_2H_5OH$-Gehalt (bis max. 2 Vol.-%) an einer Kieselgel S/$CH_2Cl_2$-Säule ($\phi$ 2,8 cm, Höhe 48 cm) chromatographiert. Nach Elution von 4,8 g Vorfraktionen wurden 3,5 g Produkt eluiert, indem die gesuchte Verbindung enthalten war. Dieses Produkt (3,5 g) wurde erneut auf die gleiche Weise wie vorstehend beschrieben an einer Kieselgel S/$CH_2Cl_2$-Säule ($\phi$ 2,0 cm, höhe 60 cm) chromatographiert, wobei man die Zusammensetzung der eluierten Fraktionen durch Dünnschichtchromatographie (DC) überprüfte. Nach Elution von 0,35 g Vorfraktionen wunden 4 Fraktionen eluiert (mit $CH_2Cl_2/C_2H_5OH$ 99,6 : 0,4 - 99,5 : 0,5), die die gesuchte Verbindung fast rein enthielten. Diese Fraktionen wurden zusammengefaßt und im Hochvakuum bei 65 °C Badtemperatur zur Gewichtskonstanz eingedampft. Man erhielt so 0,62 g (≙ 7,6 % Ausbeute) 3,3-Dimethyl-1-(1-imidazolyl)-1-phenyl-5-hexen-2-ol als zähes, farbloses Öl, das $^1$H-NMR-Spektrum bestätigte die Struktur dieser Verbindung.

| $C_{17}H_{22}N_2O$ (270,38) | | | |
|---|---|---|---|
| berechnet:<br>gefunden : | C 75,52<br>C 74,9 | H 8,20<br>H 8,3 | N 10,36 %<br>N 10,1 % |

Die Verbindung war in weiteren Chromatographiefraktionen neben unterschiedlichen Anteilen von Nebenprodukten enthalten, so daß die gebildete Menge weit über dem vorstehend angegebenen, in fast reiner Form isolierten Anteil liegt.

Beispiel 17

3,3-Dimethyl-1-(1-imidazolyl)-1-(3-methoxy-phenyl)-5 hexen-2-ol

Nach der im Beispiel 16 beschriebenen Arbeitsweise wurden 7,52 g (40 mmol) 1-(3-Methoxybenzyl)-imidazol und 4,65 g (40 mmol) TMEDA in 80 ml abs. THF mit 53,5 ml 1,5 molarer n-Butyllithium/Hexan-Lösung metalliert und mit 5,00 g 90 %igem (≙ 40 mmol) 2,2-Dimethyl-4-penten-aldehyd umgesetzt. Die Aufarbeitung erfolgte analog der im Beispiel 19. Der Extraktrückstand (12,2 g) wurde wie im Beispiel 19 beschrieben an einer Kieselgel S/$CH_2Cl_2$-Säule (ϕ 3,5 cm, Höhe 40 cm), und anschließend die vereinigten Fraktionen, die die gesuchte Verbindung enthielten (Menge 6,3 g), ein zweites Mal an einer Kieselgel S/$CH_2Cl_2$ (ϕ 2,4 cm, Höhe 88 cm)-Säule chromatographiert. Eine Fraktionsgruppe (2,5 g) der zweiten Chromatographie kristallisierte aus einer Hexan/Diisopropylether 3:1-Lösung.

Nach Isolierung der Substanz wurden 0,77 g (≙ 6,3 % Ausbeute) reines 3,3-Dimethyl-1-(1-imidazolyl)-1-(3-methoxy-phenyl)-5-hexen-2-ol vom Fp. 83 °C erhalten.

| $C_{18}H_{24}N_2O_2$ (300,41) | | | |
|---|---|---|---|
| berechnet:<br>gefunden : | C 71,97<br>C 68,9 | H 8,05<br>H 8,0 | H 9,33 %<br>N 8,6 % |

Beispiel 18

3,3-Dimethyl-1-(3-fluorphenyl)-1-(4(5)-methyl-1-imidazolyl)-5-hexen-2-ol

Eine Lösung von 4,76 g (25 mmol) 1-(3-Fluorbenzyl)-4(5)-methylimidazol (3:2 Mischung) und 2,91 g (25 mmol) TMEDA in 50 ml abs. THF wurde wie in Beispiel 19 beschrieben mit 33,4 ml 1,5 molarer n-Butyllithium/Hexan-Lösung lithiiert. Danach tropfte man bei -70 °C eine Lösung von 3,11 g 90 %igem (25 mmol) 2,2-Dimethyl-4-penten-aldehyd in 25 ml abs. THF zu, rührte 20 Min. bei ca. -70 °C nach und ließ in ca. 2,5 Stunden auf Zimmertemperatur anwärmen. Danach wurde wie im Beispiel 19 beschrieben aufgearbeitet. Den Extraktrückstand (7,6 g) chromatographierte man, wie im Beispiel 16 erläutert, an einer Kieselgel S/$CH_2Cl_2$-Säule (ϕ 2,8 cm, Höhe 48 cm). Nach Elution von 1,35 g Vorfraktionen wurden in Form von 10 Fraktionen 4,10 g Produkt eluiert, in dem die gesuchten Verbindungen angereichert vorlagen. Aus diesem Produkt wurden nach Lösen in Diethylether/Hexan kristalline Substanz erhalten, die nach Isolierung und Trocknen 2,44 g (≙ 32,3 % Ausbeute) reines 3,3-Dimethyl-1-(3-fluorphenyl)-1-(4(5)-methyl-1-imidazolyl)-5-hexen-2-ol (6,7:3,3-Mischung von 4- und 5-Methyl-imidazol-Verbindung) vom Fp. 118 °C ergab.

| $C_{18}H_{23}FN_2O$ (302,40) | | | | |
|---|---|---|---|---|
| berechnet:<br>gefunden : | C 71,49<br>C 71,3 | H 7,67<br>H 7,8 | F 6,28<br>F 6,3 | N 9,26 %<br>N 9,2 |

Beispiel 19

1-(3-Chlorphenyl)-3,3-dimethyl-1-(4-methyl-1-imidazolyl)-5-hexen-2-ol und
1-(3-Chlorphenyl)-3,3-dimethyl-1-(5-methyl-1-imidazolyl)-5-hexen-2-ol

Eine Lösung von 10,34 g (50 mmol) 1-(3-Chlor-benzyl)-4(5)-methyl-imidazol (3:2-Mischung) in 100 ml abs. THF wurde wie in Beispiel 2 beschrieben mit 33,4 ml 1,5 molarer n-Butyllithium/Hexan-Lösung lithiiert. Danach tropfte man bei -70 °C eine Lösung von 6,24 g 90 %igem (50 mmol) 2,2-Dimethyl-4-pentenal in 75 ml abs` THF zu, rührte 40 Minuten bei ca. -70 °C nach und ließ in ca. 2,5 Stdn. auf Zimmertemperatur anwärmen. Anschließend wurde wie in Beispiel 2 beschrieben aufgearbeitet. Man erhielt 1,5 g kristallines Produkt (Fp. 136-137 °C) (laut $^1$H-NMR 1-(3-Chlorphenyl)-3,3-dimethyl-1-(5(4)-methyl-1-imidazolyl)-5-hexen-2-ol; 5-Methyl:4-Methyl-Verb. 86:14) neben 11,6 g öligem Etherextrakt-Rückstand. Dieser wurde wie im Beispiel 19 beschrieben chromatographiert (Säule: $\phi$ 2,6 cm, Höhe 95 cm). Nach 1,8 g Vorfraktionen wurden Fraktionsgruppen erhalten, die laut DC und $^1$H-NMR die gesuchten Verbindungen in angereicherter Form enthalten (5,8 g). Aus diesen Fraktionsgruppen wurde jeweils aus Diethylether/Hexan-Lösungen kristallines Produkt erhalten. Aus der zuerst eluierten Fraktionsgruppe (2,33 g) erhielt man 1,35 g Kristallisat (4-Methyl:5-Methyl-Verbindung 75:25), aus der danach eluierten Gruppe 0,94 g Kristallisat (5-Methyl-: 4-Methyl-Verb. ca. 1:1). Aus der letzten Fraktionsgruppe (2,93 g) wurden 0,46 g Kristallisat, laut $^1$H-NMR reines 1-(3-Chlorphenyl)-3,3-dimethyl-1-(5-methyl-1-imidazolyl)-5-hexen-2-ol, Fp. 142-143 °C, erhalten.

| $C_{18}H_{23}ClN_2O$(318,86) | | | | |
|---|---|---|---|---|
| berechnet: | C 67,80 | H 7,27 | Cl 11,12 | N 8,79 % |
| gefunden: | C 67,5 | H 7,3 | Cl 11,0 | N 9,0 % |

Die 0,94 g Produkt (5-Methyl-: 4-Methyl-Verb. ca. 1:1) wurden aus Ethylacetat/Diethylether/Diisopropylether umkristallisiert. Dabei erhielt man 0,42 g Kristallisat mit 62 % Anteil 5-Methyl-Verbindung. Der Mutterlaugenrückstand (75 % 4-Methyl-Verb.) (0,5 g) wurde mit dem oben erhaltenen 1,35 g Produkt (75 % 4-Methyl-Verb.) vereinigt, und dieser Anteil wurde wie vorstehend erwähnt umkristallisiert. Dabei wurden 0,31 g, laut $^1$H-NMR, reines 1-(3-Chlorphenyl)-3,3-dimethyl-1-(4-methyl-1-imidazolyl)-5-hexen-2-ol, Fp. 126-127 °C, erhalten.

| $C_{18}H_{23}ClN_2O$(318,86) | | | | |
|---|---|---|---|---|
| gefunden: | C 67,6 | H 7,2 | Cl 11,0 | N 8,9 % |

Die 4-Methyl- und die 5-Methyl-1-imidazolyl-Verbindung unterscheiden sich u.a. deutlich in der Lage des $^1$H-NMR-Signals für das Proton in 2-Stellung des Imidazolrestes (gemessen in $CDCl_3$ mit TMS als internem Standard). Bei der 4-Methyl-1-imidazolyl-Verbindung liegt dieses Signal bei 7,49 $\delta$, bei der 5-Methyl-1-imidazolyl-Verbindung hingegen bei 8,16 $\delta$.

Beispiel 20

1-(4-Diphenyl)-3,3-dimethyl-1-(1-imidazolyl)-2-hexanol

Eine Lösung von 1,733 g (5 mmol) 1-(4-Diphenyl)-3,3-dimethyl-1-(1-imidazolyl)-5-hexen-2-ol (Verbindung nach Beispiel 13) in 80 ml Methanol wurde mit 170 mg 5 %igem Platin auf Aktivkohle als Katalysator versetzt und unter einer Wasserstoffatmosphäre bei schwachem Wasserstoffüberdruck bei 24 °C und 984 mbar Atmosphärendruck intensiv durchmischt. Nach 1 Stunde waren 120 ml Wasserstoff aufgenommen worden. Die Suspension wurde über Filtrierhilfe abgesaugt und das Filtrat anschließend im Vakuum eingedampft. Der verbleibende kristalline Rückstand (1,75 g) wurde kurz mit 8 ml Diisopropylether aufgekocht, im Eisbad abgekühlt, abgesaugt und getrocknet. Man erhielt 1,63 g (≙ 93,7 % Ausbeute) reines 1-(4-Diphenyl)-3,3-dimethyl-1-(1-imidazolyl)-2-hexanol vom Fp. 177 °C.

| $C_{23}H_{28}N_2O$ (348,49) | | | |
|---|---|---|---|
| berechnet:<br>gefunden : | C 79,27<br>C 78,5 | H 8,10<br>H 8,1 | N 8,04 %<br>N 7,9 % |

Beispiel 21

1-(3-Chlorphenyl)-3,3-dimethyl-1-(1-imidazolyl)-2-hexanol

In der gleichen Weise wie im Beispiel 20 beschrieben wurden durch Hydrierung von 1-(3-Chlorphenyl)-3,3-dimethyl-1-(1-imidazolyl)-5-hexen-2-ol (Verbindung nach Beispiel 2) mit 200 mg sulfidiertem 5 %igen Platin auf Aktivkohle als Katalysator im 5 mmol-Maßstab 1,43 g 1-(3-Chlorphenyl)-3,3-dimethyl-1-(1-imidazolyl)-2-hexanol vom Fp. 97° C erhalten.

| $C_{17}H_{23}ClN_2O$ (306,84) | | | | |
|---|---|---|---|---|
| berechnet:<br>gefunden : | C 66,54<br>C 66,3 | H 7,56<br>H 7,4 | Cl 11,56<br>Cl 11,7 | N 9,13 %<br>N 9,0 % |

Pharmakologische Prüfung

Prüfung auf Beeinflussung der Tetrabenazin-Ptosis Methode:

Männliche Mäuse (SPF-71, KF:NMRI), Gewicht 18-22 g, wurden randomisiert Behandlungsgruppen à 6 Tieren zugeteilt. Die Prüfsubstanzen wurden in 1 % Methylhydroxyethyl-Cellulose (MH)/Wasser suspendiert und oral in 10 ml/kg Körpergewicht verabreicht. Eine Kontrollgruppe erhielt das Lösungsmittel (1% MH), die Standardgruppe Nomifensin 10 mg/kg p.o. (in 1 % MH). Die verwendete Tetrabenazin-Lösung wurde vom Methan-Sulfonat (≙ 76,8 % Base) hergestellt und eine Menge entsprechend 40 mg/kg Tetrabenazin-Base intraperitoneal 30 Min. nach den Prüfsubstanzen appliziert. 30 Min. nach Gabe von Tetrabenazin wurde die Ptosis entsprechend der folgenden Skala beurteilt:

| Ptosis | Score |
|---|---|
| Augen geschlossen | 4 |
| Augen ¾ geschlossen | 3 |
| Augen ½ geschlossen | 2 |
| Augen ¼ geschlossen | 1 |
| Augen offen | 0 |

Die Ptosis-Scores wurden kumuliert und das Ergebnis der Gruppen auf den maximal erreichbaren Score (6 x 4 = 100 %) bezogen. Mittels linearer Regression wurde die $ED_{50}$ (mg/kg) ermittelt als die Dosis, bei der der Grad der Ptosis 50 % des bei der Kontrollgruppe ermittelten Wertes beträgt.

Folgende Ergebnisse wurden ermittelt:

| Verbindung nach Beispiel | $ED_{50}$ (Vertrauensbereich 95 %) mg/kg p.o. |
|---|---|
| 2 | 2,0 (0,5-8,8) |
| 13 | 1,3 (0,1-26,8) |
| 10 | 4,6 (2,2-9,7) |
| 1 | 3,9 (2,1-7,3) |
| 20 | 1,2 (0,3-5,4) |
| Standard Nomifensin | 1,2 (0,7-2,0) |

Die genannten Substanzen zeigten starke Wirkung im Modell der Tetrabenazin-Ptosis an der Maus, das als prädiktiv für antidepressive Wirkung beim Menschen gilt.

## Patentansprüche

### Patentansprüche für folgende Vertragsstaaten : AT, BE, CH, DE, FR, GB, IT, LI, LU, NL, SE

**1.** 1-(1-Aryl-2-hydroxyethyl)-imidazole der Formel I,

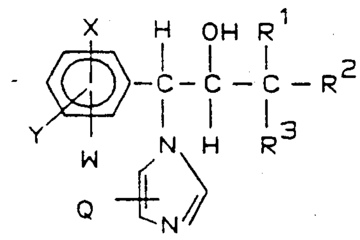

I

in der bedeuten

| | |
|---|---|
| X | H, $(C_1-C_4)$-Alkyl, Phenyl, F, Cl, Br, OH, $CF_3$, $(C_1-C_4)$-Alkoxy, $(C_1-C_4)$-Alkylthio, $-NR^4_2$ , in dem $R^4$ - gleich oder verschieden - $(C_1-C_4)$-Alkyl oder zusammen mit dem Stickstoffatom einen Pyrrolidin-, Piperidin- oder Morpholin-Rest bedeutet, |
| Y | H, $(C_1-C_4)$-Alkyl, F, Cl, Br, $(C_1-C_4)$-Alkoxy oder |
| X und Y | zusammen in 3,4-Stellung eine $-(CH_2)_L$-Kette mit L=3 oder 4, $-OCH_2CH_2-$, $-O-CH_2O-$ oder -CH=CH-CH=CH-, |
| W | H, $CH_3$ oder $OCH_3$, |
| $R^1$ | H, $(C_2-C_4)$-Alkyl, $(C_2-C_5)$-Alkenyl, $(C_2-C_5)$-Alkinyl, $(C_3-C_8)$-Cycloalkyl, $(C_4-C_8)$-Cycloalkenyl, $(C_3-C_7)$-Cycloalkyl-$CH_2$,$(C_4-C_7)$-1-Cycloalkenyl-$CH_2$, $(C_1-C_4)$-Alkoxymethyl, $(C_3-C_5)$-Alkenyloxymethyl oder 2-Propin-1-yloxymethyl, |

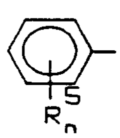 $(C_1-C_3)$-Alkyl oder 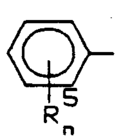 CH=CH-$CH_2$, worin

| | |
|---|---|
| $R^5$ | $CH_3$, $C_2H_5$, $OCH_3$, F, Cl, Br oder $CF_3$ und |
| n | 0,1 oder 2 bedeutet und für den Fall, daß $R^5$ $CF_3$ bedeutet, n 1 ist, |
| $R^2$ | $(C_1-C_4)$-Alkyl oder $(C_3-C_5)$-Alkenyl, |
| $R^3$ | $(C_1-C_4)$-Alkyl oder |
| $R^1$ und $R^3$ oder $R^2$ und $R^3$ | zusammen mit dem C-Atom, an dem sie gebunden sind, unsubstituiertes oder durch 1-3 $CH_3$ und/oder $OCH_3$ und/oder Cl substituiertes $(C_3-C_7)$-Cyloalkyl, $C_7$-Bicycloalkyl, $(C_4-C_7)$-Cycloalkenyl oder $C_7$-Bicycloalkenyl, wobei in den Cycloalkenyl- und Bicycloalkenyl-Resten die C,C-Doppelbindung nicht in der 1-Position steht, oder $(Oxa-C_2-C_5)$-Cycloalkyl und |
| Q | H oder $CH_3$ oder $C_2H_5$ in 4- und/oder 5-Stellung sowie deren physiologisch verträgliche Säureadditionssalze und deren Stereoisomere und optisch aktiven Enantiomere. |

**2.** Verbindungen nach Anspruch 1, dadurch gekennzeichnet, daß mindestens eines der folgenden Merkmale erfüllt ist:

| | |
|---|---|
| X | H, $(C_1-C_4)$-Alkyl, Phenyl, F, Cl, Br, OH, $(C_1-C_4)$-Alkoxy, 3-$N(CH_3)_2$ oder 3-$CF_3$, |

Y H, $CH_3$, Cl oder $OCH_3$ oder
X und Y zusammen in 3,4-Stellung eine $-(CH_2)_4-$, $-O-CH_2-O-$ oder $-CH=CH-CH=CH-$ Kette,
W H,
$R^1$ ($C_2$-$C_4$)-Alkyl, ($C_2$-$C_5$)-Alkenyl, 2-Propin-1-yl, ($C_3$-$C_6$)-Cycloalkyl, ($C_1$-$C_4$)-Alkoxymethyl, ($C_3$-$C_5$)-Alkenyloxymethyl, 2-Propin-1-yloxymethyl oder

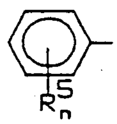
($C_1$-$C_3$)-Alkyl, worin

$R^5$ $OCH_3$, F oder Cl und n 0 oder 1 bedeutet,
$R^2$ und $R^3$ ($C_1$-$C_4$)-Alkyl oder
$R^1$ und $R^3$ oder $R^2$ und $R^3$ zusammen mit dem C-Atom, an dem sie gebunden sind, unsubstituiertes oder durch 1 oder 2 $CH_3$ und/oder $OCH_3$ substituiertes ($C_3$-$C_7$)-Cycloakyl, $C_7$-Bicycloalkyl oder $C_7$-Bicycloalkenyl, dessen C,C-Doppelbindung nicht in der 1-Position steht, oder (Oxa-$C_3$-$C_5$)-Cycloalkyl und
Q H oder $CH_3$ in 4- oder 5-Stellung.

**3.** Verbindungen nach einem der Ansprüche 1 oder 2, dadurch gekennzeichnet, daß mindestens eines der folgenden Merkmale erfüllt ist:
X H, $CH_3$, Phenyl, F, Cl, Br, 3- oder 4-$OCH_3$ oder 3-$CF_3$,
Y, H, $CH_3$, 3- oder 4-Cl oder 3- oder 4-$OCH_3$ oder
X und Y zusammen in 3,4-Stellung eine $-CH=CH-CH=CH-$Kette,
W H,
$R^1$ ($C_2$-$C_4$)-Alkyl, ($C_3$-$C_5$)-Alkenyl, 2-Propin-1-yl, Benzyl, ($C_1$-$C_4$)-Alkoxymethyl oder ($C_3$-$C_4$)-Alkenyloxymethyl,
$R^2$ und $R^3$ ($C_1$-$C_4$)-Alkyl oder
$R^1$ und $R^3$ oder $R^2$ und $R^3$ zusammen mit dem C-Atom, an dem sie gebunden sind, ($C_3$-$C_7$)-Cycloalkyl, 2-Norbornyl, Norbornen-2-yl oder (Oxa-$C_3$-$C_5$)-Cycloalkyl und
Q H oder $CH_3$ in 4- oder 5-Stellung.

**4.** Verfahren zur Herstellung von Verbindungen der Formel I gemäß Anspruch 1, dadurch gekennzeichnet, daß man
A) ein 1-Benzylimidazol der Formel IV,

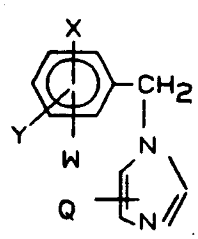
IV

worin X, Y, W und Q die in Anspruch 1 angegebenen Bedeutungen haben, mit einem oder bis zu zwei Äquivalenten einer starken Base versetzt und anschließend zuerst mit einem Aldehyd der Formel V

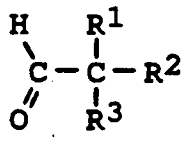

V

in der $R^1$, $R^2$ und $R^3$ die in Anspruch 1 genannten Bedeutungen haben, und danach mit einer Protonensäure, umsetzt

oder

B) ein Oxiran der Formel II,

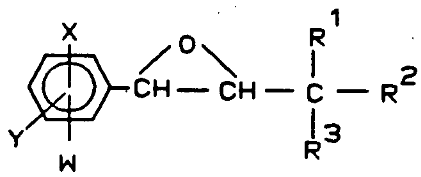

II

in der X, Y, W, $R^1$, $R^2$ und $R^3$ die in Anspruch 1 angegebenen Bedeutungen haben, durch Umsetzung mit einer Verbindung der Formel III,

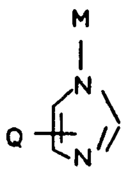

III

in der M Wasserstoff oder ein Alkali- oder Erdalkalimetall bedeutet und Q die in Anspruch 1 angegebenen Bedeutungen hat, nucleophil öffnet und dann mit einer Protonensäure versetzt,

oder

C) bei Verbindungen der Formel I, die nach Verfahren A) oder B) hergestellt worden sind, nach bekannten Methoden einen Substituenten X und/oder $R^1$ und/oder $R^2$ in einen anderen Substituenten X und/oder $R^1$ und/oder $R^2$ umwandelt, und gegebenenfalls die nach den Verfahren A), B) und/oder C) erhaltenen Verbindungen der Formel I mit anorganischen oder organischen Säuren in ihre physiologisch verträglichen Säureadditionssalze überführt und gegebenenfalls eine nach A), B) oder C) erhaltene Verbindung in ihre Stereoisomeren und/oder ihre optisch aktiven Enantiomeren auftrennt.

**5.** Verwendung einer Verbindung der Formel I nach Anspruch 1 zur Herstellung eines antimykotisch wirkenden Arzneimittels.

**6.** Arzneimittel mit antimykotischer Wirkung, gekennzeichnet durch einen wirksamen Gehalt einer Verbindung der Formel I nach Anspruch 1.

**7.** Verwendung einer Verbindung der Formel I nach Anspruch 1 zur Herstellung eines antidepressiv wirkenden Arzneimittels.

**8.** Arzneimittel mit antidepressiver Wirkung, gekennzeichnet durch einen wirksamen Gehalt einer Verbindung der Formel I nach Anspruch 1.

**Patentansprüche für folgende Vertragsstaaten : ES, GR**

**1.** Verfahren zur Herstellung von Verbindungen der Formel I

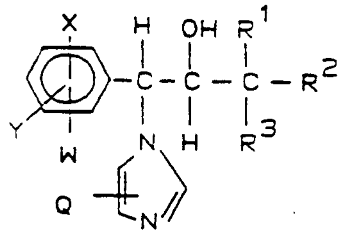

I

in der bedeuten

| | |
|---|---|
| X | H, $(C_1\text{-}C_4)$-Alkyl, Phenyl, F, Cl, Br, OH, $CF_3$, $(C_1\text{-}C_4)$-Alkoxy, $(C_1\text{-}C_4)$-Alkylthio, $-NR^4_2$ , in dem $R^4$ - gleich oder verschieden - $(C_1\text{-}C_4)$-Alkyl oder zusammen mit dem Stickstoffatom einen Pyrrolidin-, Piperidin- oder Morpholin-Rest bedeutet, |
| Y | H, $(C_1\text{-}C_4)$-Alkyl, F, Cl, Br, $(C_1\text{-}C_4)$-Alkoxy oder |
| X und Y | zusammen in 3,4-Stellung eine $-(CH_2)_L$-Kette mit L = 3 oder 4, $-OCH_2CH2-$, $-O-CH_2O-$ oder -CH = CH-CH = CH-, |
| W | H, $CH_3$ oder $OCH_3$, |
| $R^1$ | H, $(C_2\text{-}C_4)$-Alkyl, $(C_2\text{-}C_5)$-Alkenyl, $(C_2\text{-}C_5)$-Alkinyl, $(C_3\text{-}C_8)$-Cycloalkyl, $(C_4\text{-}C_8)$-Cycloalkenyl, $(C_3\text{-}C_7)$-Cycloalkyl-$CH_2$,$(C_4\text{-}C_7)$-1-Cycloalkenyl-$CH_2$, $(C_1\text{-}C_4)$-Alkoxymethyl, $(C_3\text{-}C_5)$-Alkenyloxymethyl oder 2-Propin-1-yloxymethyl, |

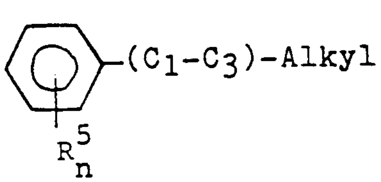

oder

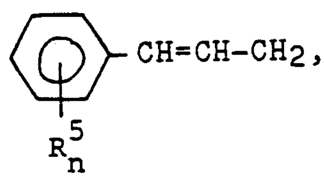

, worin

| | |
|---|---|
| $R^5$ | $CH_3$, $C_2H_5$, $OCH_3$, F, Cl, Br oder $CF_3$ und |
| n | 0,1 oder 2 bedeutet und für den Fall, daß $R^5$ $CF_3$ bedeutet, n 1 ist, |
| $R^2$ | $(C_1\text{-}C_4)$-Alkyl oder $(C_3\text{-}C_5$-Alkenyl |
| $R^3$ | $(C_1\text{-}C_4)$-Alkyl oder |
| $R^1$ und $R^3$ oder $R^2$ und $R^3$ | zusammen mit dem C-Atom, an dem sie gebunden sind, unsubstituiertes oder durch 1-3 $CH_3$ und/oder $OCH_3$ und/oder Cl substituiertes $(C_3\text{-}C_7)$-Cycloalkyl, $C_7$-Bicycloaklyl, $(C_4\text{-}C_7)$-Cycloalkenyl oder $C_7$-Bicycloalkenyl, wobei in den Cycloalkenyl- und Bicycloalkenyl-Resten die C,C-Doppelbindung nicht in der 1-Position steht, oder (Oxa-$C_2$-$C_5$)-Cycloalkyl und |
| Q | H oder $CH_3$ oder $C_2H_5$ in 4- und/oder 5-Stellung sowie deren physiologisch verträgliche Säureadditionssalze und deren Stereoisomere und optisch aktiven Enantiomere, dadurch gekennzeichnet, daß man |

A) ein 1-Benzylimidazol der Formel IV,

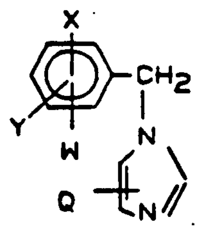

IV

worin X, Y, W und Q die in Anspruch 1 angegebenen Bedeutungen haben, mit einem oder bis zu zwei Äquivalenten einer starken Base versetzt und anschließend zuerst mit einem Aldehyd der Formel V

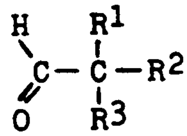

V

in der $R^1$, $R^2$ und $R^3$ die in Anspruch 1 genannten Bedeutungen haben, und danach mit einer Protonensäure, umsetzt
oder
B) ein Oxiran der Formel II,

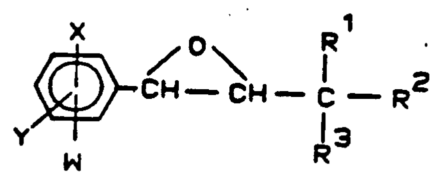

II

in der X, Y, W, $R^1$, $R^2$ und $R^3$ die in Anspruch 1 angegebenen Bedeutungen haben, durch Umsetzung mit einer Verbindung der Formel III,

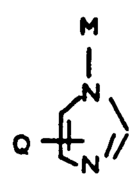

III

in der M Wasserstoff oder ein Alkali- oder Erdalkalimetall bedeutet und Q die in Anspruch 1 angegebenen Bedeutungen hat, nucleophil öffnet und dann mit einer Protonensäure versetzt,
oder
C) bei Verbindungen der Formel I, die nach Verfahren A) oder B) hergestellt worden sind, nach bekannten Methoden einen Substituenten X und/oder $R^1$ und/oder $R^2$ in einen anderen Substituenten X und/oder $R^1$ und/oder $R^2$ umwandelt, und gegebenenfalls die nach den Verfahren A), B) und/oder C) erhaltenen Verbindungen der Formel I mit anorganischen oder organischen Säuren in ihre physiologisch verträglichen Säureadditionssalze überführt und gegebenenfalls eine nach A), B) oder C) erhaltene Verbindung in ihre Stereoisomeren und/oder ihre optisch aktiven Enantiomeren auftrennt.

**2.** Verfahren nach Anspruch 1, dadurch gekennzeichnet, daß mindestens eines der folgenden Merkmale erfüllt ist:

| | |
|---|---|
| X | H, $(C_1$-$C_4)$-Alkyl, Phenyl, F, Cl, Br, OH, $(C_1$-$C_4)$-Alkoxy, 3-$N(CH_3)_2$ oder 3-$CF_3$, |
| Y | H, $CH_3$, Cl oder $OCH_3$ oder |
| | X und Y zusammen in 3,4-Stellung eine -$(CH_2)_4$-, -O-$CH_2$-O- oder -CH = CH-CH = CH- Kette, |
| W | H, |
| $R^1$ | $(C_2$-$C_4)$-Alkyl, $(C_2$-$C_5)$-Alkenyl, 2-Propin-1-yl, $(C_3$-$C_6)$-Cycloalkyl, $(C_1$-$C_4)$-Alkoxymethyl, $(C_3$-$C_5)$-Alkenyloxymethyl, 2-Propin-1-yloxymethyl oder |

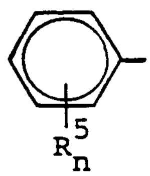 -$(C_1$-$C_3)$-Alkyl, worin

| | |
|---|---|
| $R^5$ | $OCH_3$, F oder Cl und n 0 oder 1 bedeutet, |
| $R^2$ und $R^3$ | $(C_1$-$C_4)$-Alkyl oder |
| $R^1$ und $R^3$ oder $R^2$ und $R^3$ | zusammen mit dem C-Atom, an dem sie gebunden sind, unsubstituiertes oder durch 1 oder 2 $CH_3$ und/oder $OCH_3$ substituiertes $(C_3$-$C_7)$-Cycloalkyl, $C_7$-Bicycloalkyl oder $C_7$-Bicycloalkenyl, dessen C,C-Doppelbindung nicht in der 1-Position steht, oder (Oxa-$C_3$-$C_5$)-Cycloalkyl und |
| Q | H oder $CH_3$ in 4- oder 5-Stellung. |

**3.** Verfahren nach einem der Ansprüche 1 oder 2, dadurch gekennzeichnet, daß mindestens eines der folgenden Merkmale erfüllt ist:

| | |
|---|---|
| X | H, $CH_3$, Phenyl, F, Cl, Br, 3- oder 4-$OCH_3$ oder 3-$CF_3$, |
| Y | H, $CH_3$, 3- oder 4-Cl oder 3- oder 4-$OCH_3$ oder |
| X und Y | zusammen in 3,4-Stellung eine -CH = CH-CH = CH-Kette, |
| W | H, |
| $R^1$ | $(C_2$-$C_4)$-Alkyl, $(C_3$-$C_5)$-Alkenyl, 2-Propin-1-yl, Benzyl, $(C_1$-$C_4)$-Alkoxymethyl oder $(C_3$-$C_4)$-Alkenyloxymethyl, |
| $R^2$ und $R^3$ | $(C_1$-$C_4)$-Alkyl oder |
| $R^1$ und $R^3$ oder $R^2$ und $R^3$ | zusammen mit dem C-Atom, an dem sie gebunden sind, $(C_3$-$C_7)$-Cycloalkyl, 2-Norbornyl, Norbornen-2-yl oder (Oxa-$C_3$-$C_5$)-Cycloalkyl und |
| Q | H oder $CH_3$ in 4- oder 5-Stellung. |

**4.** Verwendung einer Verbindung der Formel I erhältlich nach Anspruch 1 zur Herstellung eines antimykotisch wirkenden Arzneimittels.

**5.** Arzneimittel mit antimykotischer Wirkung, gekennzeichnet durch einen wirksamen Gehalt einer Verbindung der Formel I erhältlich nach Anspruch 1.

**6.** Verwendung einer Verbindung der Formel I erhältlich nach Anspruch 1 zur Herstellung eines antidepressiv wirkenden Arzneimittels.

**7.** Arzneimittel mit antidepressiver Wirkung, gekennzeichnet durch einen wirksamen Gehalt einer Verbindung der Formel I erhältlich nach Anspruch 1.

## Claims

**Claims for the following Contracting States : AT, BE, CH, DE, FR, GB, IT, LI, LU, NL, SE**

**1.** 1-(1-Aryl-2-hydroxyethyl)-imidazole of the formula I

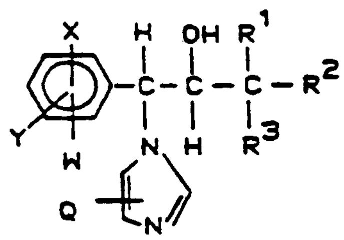

in which

| | |
|---|---|
| X | denotes B, $(C_1-C_4)$-alkyl, phenyl, F, Cl, Br, OH, $CF_3$, $(C_1-C_4)$-alkoxy, $(C_1-C_4)$-alkylthio or $-NR^4_2$ , in which $R^4$ are identical or different and denote $(C_1-C_4)$-alkyl or, together with the nitrogen atom, a pyrrolidine, piperidine or morpholine radical, |
| Y | denotes H, $(C_1-C_4)$-alkyl, F, Cl, Br or $(C_1-C_4)$-alkoxy, or |
| X and Y | together in the 3,4-position denote a $-(CH_2)_L$-chain, where L = 3 or 4, $-OCH_2CH_2-$, $-O-CH_2O-$ or $-CH=CH-CH=CH-$, |
| W | denotes H, $CH_3$ or $OCH_3$, |
| $R^1$ | denotes H, $(C_2-C_4)$-alkyl, $(C_2-C_5)$-alkenyl, $(C_2-C_5)$alkynyl, $(C_3-C_8)$-cycloalkyl, $(C_4-C_8)$-cycloalkenyl, $(C_3-C_7)$-cycloalkyl-$CH_2$, $(C_4-C_7)$-1-cycloalkenyl-$CH_2$, $(C_1-C_4)$-alkoxymethyl, $(C_3-C_5)$-alkenyloxymethyl or 2-propyn-1-yloxymethyl, |

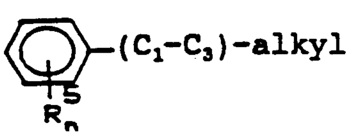 or 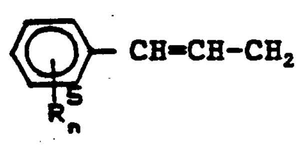 in which

| | |
|---|---|
| $R^5$ | denotes $CH_3$, $C_2H_5$, $OCH_3$, F, Cl, Br or $CF_3$, and |
| n | denotes 0, 1 or 2 and, in the case where $R^5$ denotes $CF_3$, n is 1, |
| $R^2$ | denotes $(C_1-C_4)$-alkyl or $(C_3-C_5)$-alkenyl, |
| $R^3$ | denotes $(C_1-C_4)$-alkyl or |
| $R^1$ and $R^3$ or $R^2$ and $R^3$, | together with the carbon atom to which they are bound, denote $(C_3-C_7)$-cycloalkyl, $C_7$-bicycloalkyl, $(C_4-C_7)$-cycloalkenyl or $C_7$-bicycloalkenyl which is in each case unsubstituted or substituted by 1-3 $CH_3$ and/or $OCH_3$ and/or Cl and in which, in cycloalkenyl and bicycloalkenyl radicals, the C=C double bond is not in the 1-position, or (oxa-$C_2-C_5$)-cycloalkyl, and |
| Q | denotes H or $CH_3$ or $C_2H_5$ in the 4- and/or 5-position, and the physiologically acceptable acid-addition salts thereof and the stereoisomers and optically active enantiomers thereof. |

**2.** A compound as claimed in claim 1, wherein at least one of the following features is fulfilled:

| | |
|---|---|
| X | denotes H, $(C_1-C_4)$-alkyl, phenyl, F, Cl, Br, OH, $(C_1-C_4)$-alkoxy, 3-N-$(CH_3)_2$ or 3-$CF_3$, |
| Y | denotes H, $CH_3$, Cl or $OCH_3$, or |
| | X and Y together in the 3,4-position denote a $-(CH_2)_4-$, $-O-CH_2-O-$ or $-CH=CH-CH=CH-$ chain, |
| W | denotes H, |
| $R^1$ | denotes $(C_2-C_4)$-alkyl, $(C_2-C_5)$-alkenyl, 2-propyn-1-yl, $(C_3-C_6)$-cycloalkyl, |

($C_1$-$C_4$)-alkoxymethyl,
($C_3$-$C_5$)-alkenyloxymethyl, 2-propyn-1-yloxymethyl or

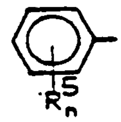 ($C_1$-$C_3$)-alkyl, in which

| | |
|---|---|
| $R^5$ | denotes $OCH_3$, F or Cl and n denotes 0 or 1, |
| $R^2$ and $R^3$ | denote ($C_1$-$C_4$)alkyl or |
| $R^1$ and $R^3$ or $R^2$ and $R^3$, | together with the carbon atom to which they are bound, denote ($C_3$-$C_7$)-cycloalkyl, $C_7$-bicycloalkyl or $C_7$-bicycloalkenyl whose C=C double bond is not in the 1-position, each of which is unsubstituted or substituted by 1 or 2 $CH_3$ and/or $OCH_3$, or (oxa-$C_3$-$C_5$)-cycloalkyl, and |
| Q | denotes H or $CH_3$ in the 4- or 5-position. |

**3.** A compound as claimed in any one of claims 1 or 2, wherein at least one of the following features is fulfilled:

| | |
|---|---|
| X | denotes H, $CH_3$, phenyl, F, Cl, Br, 3- or 4-$OCH_3$ or 3-$CF_3$, |
| Y | denotes H, $CH_3$, 3- or 4-Cl or 3- or 4-$OCH_3$, or |
| X and Y | together in the 3,4-position denote a -CH=CH-CH=CH-chain, |
| W | denotes H, |
| $R^1$ | denotes ($C_2$-$C_4$)-alkyl, ($C_3$-$C_5$)-alkenyl, 2-propyn-1-yl, benzyl, ($C_1$-C4)-alkoxymethyl, or ($C_3$-$C_4$)-alkenyloxymethyl, |
| $R^2$ and $R^3$ | denote ($C_1$-$C_4$)-alkyl or |
| $R^1$ and $R^3$ or $R^2$ and $R^3$, | together with the carbon atom to which they are bound, denote ($C_3$-$C_7$)-cycloalkyl, 2-norbornyl, norbornen-2-yl or (oxa-$C_3$-$C_5$)-cycloalkyl, and |
| Q | denotes H or $CH_3$ in the 4- or 5-position. |

**4.** A process for the preparation of a compound of the formula I as claimed in claim 1, which comprises
A) adding one or up to two equivalents of a strong, base to a 1-benzylimidazole of the formula IV

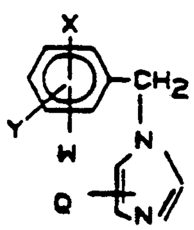

IV

in which X, Y, W and Q have the meanings mentioned in claim 1,and subsequently reacting the mixture initially with an aldehyde of the formula V

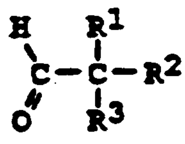

V

in which $R^1$, $R^2$ and $R^3$ have the meanings mentioned in claim 1, and then with a protonic acid,
or

B) opening an oxirane of the formula II

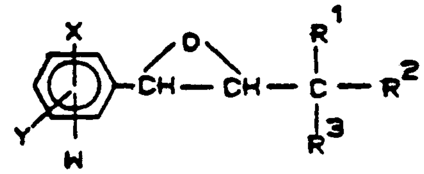

II

in which X, Y, W, $R^1$, $R^2$ and $R^3$ have the meanings mentioned in claim 1, nucleophilically through reaction with a compound of the formula III

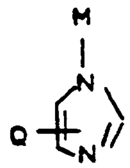

III

in which M denotes hydrogen or an alkali metal or alkaline-earth metal, and Q has the meanings mentioned in claim 1, and then adding a protonic acid,
or
C) in the case of compounds of the formula I which have been prepared by process A) or B), converting a substituent X and/or $R^1$ and/or $R^2$ into another substituent X and/or $R^1$ and/or $R^2$ by known methods, and converting the compounds of the formula I obtained by processes A), B) and/or C), if appropriate, into their physiologically acceptable acid-addition salts using inorganic or organic acids, and resolving a compound obtained by A), B) or C), if appropriate, into its stereoisomers and/or its optically active enantiomers.

5. The use of a compound of the formula I as claimed in claim 1 for the preparation of a medicament having an antimycotic action.

6. A medicament having an antimycotic action, containing an active content of a compound of the formula I as claimed in claim 1.

7. The use of a compound of the formula I as claimed in claim 1 for the preparation of a medicament having an antidepressive action.

8. A medicament having an antidepressive action, containing an active content of a compound of the formula I as claimed in claim 1.

**Claims for the following Contracting States : ES, GR**

1. A process for the preparation of a compound of the formula I

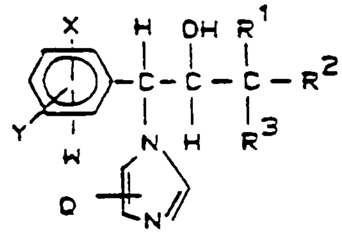

I

in which

X denotes H, $(C_1-C_4)$-alkyl, phenyl, F, Cl, Br, OH, $CF_3$, $(C_1-C_4)$-alkoxy, $(C_1-C_4)$-alkylthio or $-NR^4_2$, in which $R^4$ are identical or different and denote $(C_1-C_4)$-alkyl or, together with the nitrogen atom, a pyrrolidine, piperidine or morpholine radical,

Y denotes H, $(C_1-C_4)$-alkyl, F, Cl, Br or $(C_1-C_4)$-alkoxy, or

X and Y together in the 3,4-position denote a $-(CH_2)_L$-chain, where L = 3 or 4, $-OCH_2CH_2-$, $-O-CH_2O-$ or $-CH=CH-CH=CH$,

W denotes H, $CH_3$ or $OCH_3$,

$R^1$ denotes H, $(C_2-C_4)$-alkyl, $(C_2-C_5)$-alkenyl, $(C_2-C_5)$ alkynyl, $(C_3-C_8)$-cycloalkyl, $(C_4-C_8)$-cycloalkenyl, $(C_3-C_7)$-cycloalkyl-$CH_2$, $(C_4-C_7)$-1-cycloalkenyl-$CH_2$, $(C_1-C_4)$-alkoxymethyl, $(C_3-C_5)$-alkenyloxymethyl or 2-propyn-1-yloxymethyl,

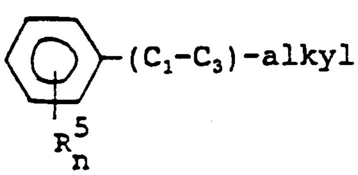 or 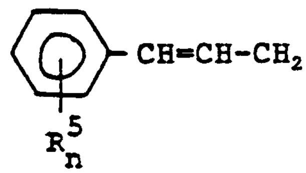 in which

$R^5$ denotes $CH_3$, $C_2H_5$, $OCH_3$, F, Cl, Br or $CF_3$, and

n denotes 0, 1 or 2 and, in the case where $R^5$ denotes $CF_3$, n is 1,

$R^2$ denotes $(C_1-C_4)$-alkyl or $(C_3-C_5)$-alkenyl,

$R^3$ denotes $(C_1-C_4)$-alkyl or

$R^1$ and $R^3$ or $R^2$ and $R^3$, together with the carbon atom to which they are bound, denote $(C_3-C_7)$-cycloalkyl, $C_7$-bicycloalkyl, $(C_4-C_7)$-cycloalkenyl or $C_7$-bicycloalkenyl which is in each case unsubstituted or substituted by 1-3 $CH_3$ and/or $OCH_3$ and/or Cl and in which, in cycloalkenyl and bicycloalkenyl radicals, the C=C double bond is not in the 1-position, or (oxa-$C_2-C_5$)-cycloalkyl, and

Q denotes H or $CH_3$ or $C_2H_5$ in the 4- and/or 5-position, and the physiologically acceptable acid-addition salts thereof and the stereoisomers and optically active enantiomers thereof which comprises

A) adding one or up to two equivalents of a strong base to a 1-benzylimidazole of the formula IV

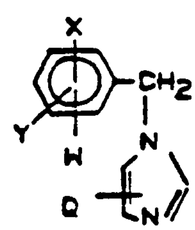

IV

in which X, Y, W and Q have the meanings mentioned in claim 1, and subsequently reacting the mixture initially with an aldehyde of the formula V

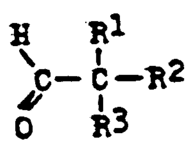

V

in which $R^1$, $R^2$ and $R^3$ have the meanings mentioned in claim 1, and then with a protonic acid, or

B) opening an oxirane of the formula II

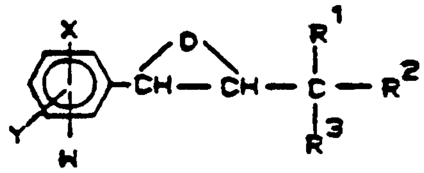

II

in which X, Y, W, $R^1$, $R^2$ and $R^3$ have the meanings mentioned in claim 1, nucleophilically through reaction with a compound of the formula III

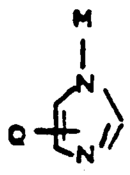

III

in which M denotes hydrogen or an alkali metal or alkaline-earth metal, and Q has the meanings mentioned in claim 1, and then adding a protonic acid,
or
C) in the case of compounds of the formula I which have been prepared by process A) or B), converting a substituent X and/or $R^1$ and/or $R^2$ into another substituent X and/or $R^1$ and/or $R^2$ by known methods, and converting the compounds of the formula I obtained by processes A), B) and/or C), if appropriate, into their physiologically acceptable acid-addition salts using inorganic or organic acids, and resolving a compound obtained by A), B) or C), if appropriate, into its stereoisomers and/or its optically active enantiomers.

**2.** A process as claimed in claim 1, wherein at least one of the following features is fulfilled:

| | |
|---|---|
| X | denotes H, $(C_1-C_4)$-alkyl, phenyl, F, Cl, Br, OH, $(C_1-C_4)$-alkoxy, 3-N-$(CH_3)_2$ or 3-$CF_3$, |
| Y | denotes H, $CH_3$, Cl or $OCH_3$, or |
| | X and Y together in the 3,4-position denote a $-(CH_2)_4-$, $-O-CH_2-O-$ or $-CH=CH-CH=CH-$ chain, |
| W | denotes H, |
| $R^1$ | denotes $(C_2-C_4)$-alkyl, $(C_2-C_5)$-alkenyl, 2-propyn-1-yl, $(C_3-C_6)$-cycloalkyl, $(C_1-C_4)$-alkoxymethyl, $(C_3-C_5)$-alkenyloxymethyl, 2-propyn-1-yloxymethyl or |

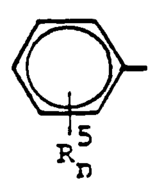
$(C_1-C_3)$-alkyl, in which

| | |
|---|---|
| $R^5$ | denotes $OCH_3$, F or Cl and n denotes 0 or 1, |
| $R^2$ and $R^3$ | denote $(C_1-C_4)$alkyl or |
| $R^1$ and $R^3$ or $R^2$ and $R^3$, | together with the carbon atom to which they are bound, denote $(C_3-C_7)$-cycloalkyl, $C_7$-bicycloalkyl or $C_7$-bicycloalkenyl whose C=C double bond is not in the 1-position, each of which is unsubstituted or substituted by 1 or 2 $CH_3$ and/or $OCH_3$, or (oxa-$C_3$-$C_5$)-cycloalkyl, and |
| Q | denotes H or $CH_3$ in the 4- or 5-position. |

**3.** A process as claimed in any one of claims 1 or 2, wherein at least one of the following features is fulfilled:

| | |
|---|---|
| X | denotes H, $CH_3$, phenyl, F, Cl, Br, 3- or 4-$OCH_3$ or 3-$CF_3$, |

| | |
|---|---|
| Y | denotes H, $CH_3$, 3- or 4-Cl or 3- or 4-$OCH_3$, or |
| X and Y | together in the 3,4-position denote a -CH = CH-CH = CH-chain, |
| W | denotes H, |
| $R^1$ | denotes ($C_2$-$C_4$)-alkyl, ($C_3$-$C_5$)-alkenyl, 2-propyn-1-yl, benzyl, ($C_1$-C4)-alkoxymethyl, or ($C_3$-$C_4$)-alkenyloxymethyl, |
| $R^2$ and $R^3$ | denote ($C_1$-$C_4$)-alkyl or |
| $R^1$ and $R^3$ or $R^2$ and $R^3$, | together with the carbon atom to which they are bound, denote ($C_3$-$C_7$)-cycloalkyl, 2-norbornyl, norbornen-2-yl or (oxa-$C_3$-$C_5$)-cycloalkyl, and |
| Q | denotes H or $CH_3$ in the 4- or 5-position. |

**4.** The use of a compound of the formula I obtainable as claimed in claim 1 for the preparation of a medicament having an antimycotic action.

**5.** A medicament having an antimycotic action, containing an active content of a compound of the formula I obtainable as claimed in claim 1.

**6.** The use of a compound of the formula I obtainable as claimed in claim 1 for the preparation of a medicament having an antidepressive action.

**7.** A medicament having an antidepressive action, containing an active content of a compound of the formula I obtainable as claimed in claim 1.

**Revendications**

**Revendications pour les Etats contractants suivants : AT, BE, CH, DE, FR, GB, IT, LI, LU, NL, SE**

**1.** 1-(1-aryl-2-hydroxyéthyl)-imidazoles de formule I

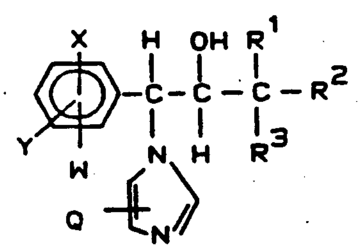

I

dans laquelle

| | |
|---|---|
| X | représente H, un groupe alkyle en $C_1$-$C_4$, phényle, F, Cl, Br, OH, $CF_3$, alcoxy en $C_1$-$C_4$, alkyl($C_1$-$C_4$)-thio ou un groupe -$NR^4{}_2$, dans lequel les radicaux $R^4$ - identiques ou différents - représentent des radicaux alkyle en $C_1$-$C_4$ ou, conjointement avec l'atome d'azote, forment un reste pyrrolidino, pipéridino ou morpholino, |
| Y | représente H, F, Cl, Br ou un groupe alkyle en $C_1$-$C_4$ ou alcoxy en $C_1$-$C_4$, ou |
| X et Y | forment ensemble en position 3,4 une chaîne -$(CH_2)_L$-avec L = 3 ou 4, -$OCH_2CH_2$-, -O-$CH_2$O- ou -CH = CH-CH = CH-, |
| W | représente H, $CH_3$ ou $OCH_3$, |
| $R^1$ | représente H ou un groupe alkyle en $C_2$-$C_4$, alcényle en $C_2$-$C_5$, alcynyle en $C_2$-$C_5$, cycloalkyle en $C_3$-$C_8$, cycloalcényle en $C_4$-$C_8$, cycloalkyl($C_3$-$C_7$)-$CH_2$, 1-cycloalcényl-($C_4$-$C_7$)-$CH_2$, alcoxy($C_1$-$C_4$)-méthyle, alcényloxy-($C_3$-$C_5$)-méthyle ou 2-propin-1-yloxyméthyle, |

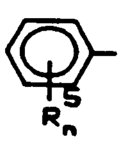

alkyle($C_1$-$C_3$) ou

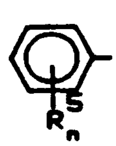

$CH{=}CH{-}CH_2$,

$R^5$ étant $CH_3$, $C_2H_5$, $OCH_3$, F, Cl, Br ou $CF_3$ et

n étant 0, 1 ou 2, et dans le cas où $R^5$ représente $CF_3$, n étant 1,

$R^2$ représente un groupe alkyle en $C_1$-$C_4$ ou alcényle en $C_3$-$C_5$,

$R^3$ représente un groupe alkyle en $C_1$-$C_4$, ou

$R^1$ et $R^3$, ou $R^2$ et $R^3$, conjointement avec l'atome de carbone auquel ils sont liés, représentent un radical cycloalkyle en $C_3$-$C_7$, bicycloalkyle en $C_7$, cycloalcényle en $C_4$-$C_7$ ou bicycloalcényle en $C_7$, non substitués ou substitués par 1-3 groupes $CH_3$ et/ou $OCH_3$ et/ou atomes de Cl, la double liaison C-C n'étant pas en position 1 dans les radicaux cycloalcényle et bicycloalcényle, ou un radical oxa-cycloalkyle($C_2$-$C_5$) et

Q représente H ou $CH_3$ ou $C_2H_5$ en position 4 et/ou 5,

ainsi que leurs sels d'addition avec des acides physiologiquement acceptables et leurs stéréoisomères et énantiomères optiquement actifs.

**2.** Composés selon la revendication 1, caractérisés en ce que s'applique au moins l'une des caractéristiques suivantes:

X représente H ou un groupe alkyle en $C_1$-$C_4$, phényle, F, Cl, Br, OH, alcoxy en $C_1$-$C_4$, 3-$N(CH_3)_2$ ou 3-$CF_3$,

Y représente H, $CH_3$, Cl ou $OCH_3$, ou

X et Y forment ensemble en position 3,4 une chaîne $-(CH_2)_4-$, $-O-CH_2-O-$ ou $CH=CH-CH=CH-$,

W représente H,

$R^1$ représente un radical alkyle en $C_2$-$C_4$, alcényle en $C_2$-$C_5$, 2-propin-1-yle, cycloalkyle en $C_3$-$C_6$, alcoxy($C_1$-$C_4$)-méthyle, alcényloxy($C_3$-$C_5$)-méthyle, 2-propin-1-yloxyméthyle ou

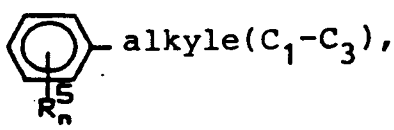

$R^5$ représentant $OCH_3$, F ou Cl et n étant 0 ou 1,

$R^2$ et $R^3$ représentent un groupe alkyle en $C_1$-$C_4$, ou

$R^1$ et $R^3$ ou $R^2$ et $R^3$, conjointement avec l'atome de carbone auquel ils sont liés, représentent un radical cycloalkyle en $C_3$-$C_7$, bicycloalkyle en $C_7$ ou bicycloalcényle en $C_7$ non substitués ou substitués par 1 ou 2 groupes $CH_3$ et/ou $OCH_3$, dont la double liaison C-C n'est pas en position 1, ou un radical oxa-cycloalkyle en $C_3$-$C_5$, et

Q représente H ou $CH_3$ en position 4 ou 5.

**3.** Composés selon la revendication 1 ou 2, caractérisés en ce que s'applique au moins l'une des caractéristiques suivantes:

X représente H, $CH_3$, le groupe phényle, F, Cl, Br, 3- ou 4-$OCH_3$ ou 3-$CF_3$,

Y représente H, $CH_3$, 3- ou 4-Cl ou 3- ou 4-$OCH_3$, ou

X et Y forment ensemble en position 3,4 une chaîne $-CH=CH-CH=CH-$,

W est H,

$R^1$ représente un groupe alkyle en $C_2$-$C_4$, alcényle en $C_3$-$C_5$, 2-propin-1-yle, benzyle, alcoxy($C_1$-$C_4$)-méthyle ou alcényloxy($C_3$-$C_4$)-méthyle,

$R^2$ et $R^3$ représentent un groupe alkyle en $C_1$-$C_4$ ou

$R^1$ et $R^3$ ou $R^2$ et $R^3$, conjointement avec l'atome de carbone auquel ils sont liés, forment un radical cycloalkyle en $C_3$-$C_7$, 2-norbornyle, norbornène-2-yle ou oxa-cycloalkyle($C_3$-$C_5$), et

Q représente H ou $CH_3$ en position 4 ou 5.

**4.** Procédé pour la préparation de composés de formule I selon la revendication 1, caractérisé en ce que

A) on ajoute de 1 à 2 équivalents d'une base forte à un 1-benzylimidazole de formule IV

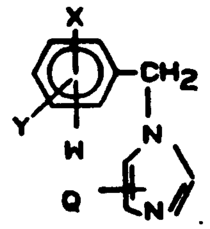

IV

dans laquelle X, Y, W et Q ont les significations données dans la revendication 1, et ensuite on le fait réagir d'abord avec un aldéhyde de formule V

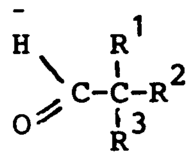

V

dans laquelle $R^1$, $R^2$ et $R^3$ ont les significations données dans la revendication 1, et ensuite avec un acide protonique, ou

B) on soumet à une ouverture nucléophile un oxiranne de formule II

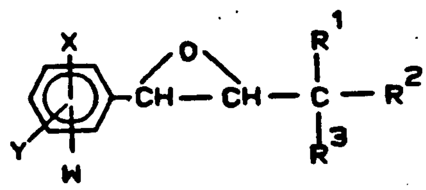

II

dans laquelle X, Y, W, $R^1$, $R^2$ et $R^3$ ont les significations données dans la revendication 1, par réaction avec un composé de formule III

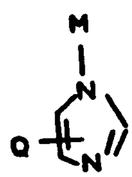

III

dans laquelle M représente un atome d'hydrogène ou d'un métal alcalin ou alcalino-terreux et Q a la signification donnée dans la revendication 1, et on lui ajoute ensuite un acide protonique, ou

C) on transforme dans les composés de formule I, qui ont été préparés par le procédé A) ou a), selon des méthodes connues, un substituant X et/ou $R^1$ et/ou $R^2$ en un autre substituant X et/ou $R^1$ et/ou $R^2$, et éventuellement on convertit en leurs sels d'addition avec des acides physiologiquement acceptables les composés de formule I, obtenus selon le procédé A), B) et/ou C), à l'aide d'acides organiques ou minéraux, et éventuellement on dédouble un composé obtenu selon A), B) ou C) en ses stéréoisomères et/ou énantiomères optiquement actifs.

5. Utilisation d'un composé de formule I selon la revendication 1, pour la préparation d'un médicament à action antimycosique.

6. Médicament à action antimycosique, caractérisé par une teneur efficace en un composé de formule I selon la revendication 1.

**7.** Utilisation d'un composé de formule I selon la revendication 1, pour la fabrication d'un médicament à action antidépressive.

**8.** Médicament à action antidépressive, caractérisé par une teneur efficace en un composé de formule I selon la revendication 1.

**Revendications pour les Etats contractants suivants : ES, GR**

**1.** Procédé pour la préparation de composés de formule I

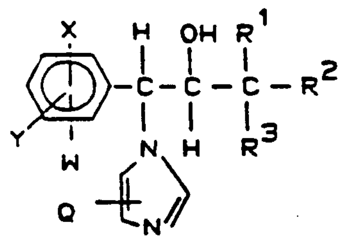

I

dans laquelle

X représente H, un groupe alkyle en $C_1$-$C_4$, phényle, F, Cl, Br, OH, $CF_3$, alcoxy en $C_1$-$C_4$, alkyl($C_1$-$C_4$)-thio ou un groupe $-NR^4{}_2$, dans lequel les radicaux $R^4$ - identiques ou différents - représentent des radicaux alkyle en $C_1$-$C_4$ ou, conjointement avec l'atome d'azote, forment un reste pyrrolidino, pipéridino ou morpholino,

Y représente H, F, Cl, Br ou un groupe alkyle en $C_1$-$C_4$ ou alcoxy en $C_1$-$C_4$, ou

X et Y forment ensemble en position 3,4 une chaîne $-(CH_2)_L$-avec L = 3 ou 4, $-OCH_2CH_2-$, $-O-CH_2O-$ ou -CH = CH-CH = CH-,

W représente H, $CH_3$ ou $OCH_3$,

$R^1$ représente H ou un groupe alkyle en $C_2$-$C_4$, alcényle en $C_2$-$C_5$, alcynyle en $C_2$-$C_5$, cycloalkyle en $C_3$-$C_8$, cycloalcényle en $C_4$-$C_8$, cycloalkyl($C_3$-$C_7$)-$CH_2$, 1-cycloalcényl-($C_4$-$C_7$)-$CH_2$, alcoxy($C_1$-$C_4$)-méthyle, alcényloxy-($C_3$-$C_5$)-méthyle ou 2-propin-1-yloxyméthyle,

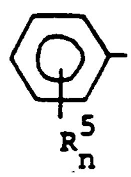 alkyle($C_1$-$C_3$) ou 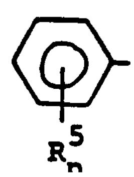 CH=CH-$CH_2$,

$R^5$ étant $CH_3$, $C_2H_5$, $OCH_3$, F, Cl, Br ou $CF_3$ et

n étant 0, 1 ou 2, et dans le cas ou $R^5$ représente $CF_3$, n étant 1,

$R^2$ représente un groupe alkyle en $C_1$-$C_4$ ou alcényle en $C_3$-$C_5$,

$R^3$ représente un groupe alkyle en $C_1$-$C_4$, ou

$R^1$ et $R^3$, ou $R^2$ et $R^3$, conjointement avec l'atome de carbone auquel ils sont liés, représentent un radical cycloalkyle en $C_3$-$C_7$, bicycloalkyle en $C_7$, cycloalcényle en $C_4$-$C_7$ ou bicycloalcényle en $C_7$, non substitués ou substitués par 1-3 groupes $CH_3$ et/ou $OCH_3$ et/ou atomes de Cl, la double liaison C-C n'étant pas en position 1 dans les radicaux cycloalcényle et bicycloalcényle, ou un radical oxa-cycloalkyle($C_2$-$C_5$) et

Q représente H ou $CH_3$ ou $C_2H_5$ en position 4 et/ou 5,

ainsi que de leurs sels d'addition avec des acides physiologiquement acceptables et de leurs stéréoisomères et énantiomères optiquement actifs, caractérisé en ce que

A) on ajoute de 1 à 2 équivalents d'une base forte à un 1-benzylimidazole de formule IV

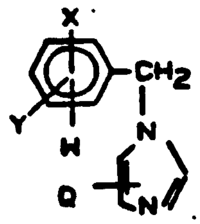

IV

dans laquelle X, Y, W et Q ont les significations données plus haut, et ensuite on le fait réagir d'abord avec un aldéhyde de formule V

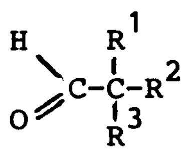

V

dans laquelle $R^1$, $R^2$ et $R^3$ ont les significations données plus haut, et ensuite avec un acide protonique, ou

B) on soumet à une ouverture nucléophile un oxiranne de formule II

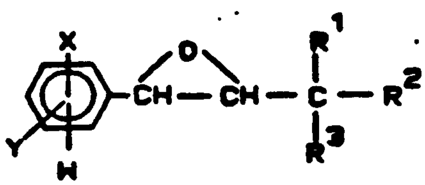

II

dans laquelle X, Y, W, $R^1$, $R^2$ et $R^3$ ont les significations données plus haut, par réaction avec un composé de formule III

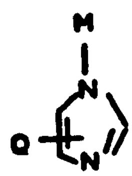

III

dans laquelle M représente un atome d'hydrogène ou d'un métal alcalin ou alcalino-terreux et Q a la significations données plus haut, et on lui ajoute ensuite un acide protonique, ou

C) on transforme dans les composés de formule I, qui ont été préparés par le procédé A) ou B), selon des méthodes connues, un substituant X et/ou $R^1$ et/ou $R^2$ en un autre substituant X et/ou $R^1$ et/ou $R^2$, et éventuellement on convertit en leurs sels d'addition avec des acides physiologiquement acceptables les composés de formule I, obtenus selon le procédé A), B) et/ou C), à l'aide d'acides organiques ou minéraux, et éventuellement on dédouble un composé obtenu selon A), B) ou C) en ses stéréoisomères et/ou énantiomères optiquement actifs.

**2.** Procédé selon la revendication 1, caractérisé en ce que s'applique au moins l'une des caractéristiques suivantes:

| | |
|---|---|
| X | représente H ou un groupe alkyle en $C_1$-$C_4$, phényle, F, Cl, Br, OH, alcoxy en $C_1$-$C_4$, 3-$N(CH_3)_2$ ou 3-$CF_3$, |
| Y | représente H, $CH_3$, Cl ou $OCH_3$, ou |
| X et Y | forment ensemble en position 3,4 une chaîne $-(CH_2)_4-$, $-O-CH_2-O-$ ou $CH=CH-CH=CH-$, |
| W | représente H, |

$R^1$ représente un radical alkyle en $C_2$-$C_4$, alcényle en $C_2$-$C_5$, 2-propin-1-yle, cycloalkyle en $C_3$-$C_6$, alcoxy($C_1$-$C_4$)-méthyle, alcényloxy($C_3$-$C_5$)-méthyle, 2-propin-1-yloxy méthyle ou

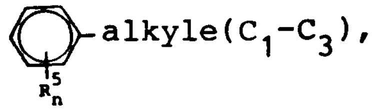
-alkyle($C_1$-$C_3$),

$R^5$ représentant $OCH_3$, F ou Cl et n étant 0 ou 1,

$R^2$ et $R^3$ représentent un groupe alkyle en $C_1$-$C_4$, ou

$R^1$ et $R^3$ ou $R^2$ et $R^3$, conjointement avec l'atome de carbone auquel ils sont liés, représentent un radical cycloalkyle en $C_3$-$C_7$, bicycloalkyle en $C_7$ ou bicycloalcényle en $C_7$ non substitués ou substitués par 1 ou 2 groupes $CH_3$ et/ou $OCH_3$, dont la double liaison C-C n'est pas en position 1, ou un radical oxa-cycloalkyle en $C_3$-$C_5$, et

Q représente H ou $CH_3$ en position 4 ou 5.

**3.** Procédé selon la revendication 1 ou 2, caractérisé en ce que s'applique au moins l'une des caractéristiques suivantes:

X représente H, $CH_3$, le groupe phényle, F, Cl, Br, 3- ou 4-$OCH_3$ ou 3-$CF_3$,

Y représente H, $CH_3$, 3- ou 4-Cl ou 3- ou 4-$OCH_3$, ou

X et Y forment ensemble en position 3,4 une chaîne -CH = CH-CH = CH-,

W est H,

$R^1$ représente un groupe alkyle en $C_2$-$C_4$, alcényle en $C_3$-$C_5$, 2-propin-1-yle, benzyle, alcoxy($C_1$-$C_4$)-méthyle ou alcényloxy($C_3$-$C_4$)-méthyle,

$R^2$ et $R^3$ représentent un groupe alkyle en $C_1$-$C_4$ ou

$R^1$ et $R^3$ ou $R^2$ et $R^3$, conjointement avec l'atome de carbone auquel ils sont liés, forment un radical cycloalkyle en $C_3$-$C_7$, 2-norbornyle, norbornène-2-yle ou oxa-cycloalkyle($C_3$-$C_5$), et

Q représente H ou $CH_3$ en position 4 ou 5.

**4.** Utilisation d'un composé de formule I préparable selon la revendication 1, pour la fabrication d'un médicament à action antimycosique.

**5.** Médicament à action antimycosique, caractérisé par une teneur efficace en un composé de formule I préparable selon la revendication 1.

**6.** Utilisation d'un composé de formule I préparable selon la revendication 1, pour la fabrication d'un médicament à action antidépressive.

**7.** Médicament à action antidépressive, caractérisé par une teneur efficace en un composé de formule I préparable selon la revendication 1.